# EUROPEAN PATENT APPLICATION

(11) **EP 4 442 358 A2**
(43) Date of publication of application: **09.10.2024**
(21) Application number: 24164270.1
(22) Date of filing: 18.03.2024
(51) Int. Cl.: B01J 13/04

(54) **MULTILAYERED CAPSULE WITH CONTROLLABLE ARRANGEMENT OF LAYERS AND INDUSTRIAL SYSTEMS AND PROCESSES OF FABRICATING THE SAME**

(30) Priority: 19.03.2023 IL 30148123
(71) Applicant: Capsule Minimal Ltd., 6492105 En Hanaziv (IL)
(72) Inventor: KRUPIK, Amichai, 4640525 Herzeliya (IL); GOETHE, Yael, 1080500 Kibbutz Ein Ha' Naziv (IL)
(74) Representative: Pearl Cohen Zedek Latzer Baratz UK LLP

(57) **Abstract**

A dissolvable multilayered capsule, for preparation of cosmetic products, with controllable arrangement of layers, having an impact effect on the dissolvability and/or mixing thereof, based on predetermined interaction between different ingredients incorporated in different layers of the capsule, is described; the method includes: providing a first substance and second substance; determining an impact effect of an interim solution of the first substance, on dissolution and/or mixing of the second substance; defining a desired target effect on dissolution and/or mixing of the multilayered capsule; setting a prioritizing preference for dissolving the first substance before the second substance, based on the impact effect and the desired target effect, and fabricating a multilayered capsule by forming a discreet inner layer and outer layer essentially covering the inner layer, where the first substance is incorporated either within the inner or outer layer discreet layer, based on the prioritizing preference.

## Description

### TECHNICAL FIELD

In general, the present invention pertains to the art of industrial chemistry and environmentally sustainable consumer chemical products. In particular, the invention relates to a readily dissolvable multilayered capsule with controllable arrangement of layers, expediting the dissolvability rate thereof, as well as to industrial systems and processes of fabricating the same.

### BACKGROUND ART

It is believed that the current state of the art is represented by the following patent literature: US5672359, US6727215, US2015275153, US2018282672, US2020308517, US2021163860, US20230149267, EP1601754, EP3596194, EP4048720 and GB2365018.

US6727215 discloses an article comprising a first pouch made of a water-reactive material, which comprises a first solid or liquid composition; and a second pouch made of a water-reactive material, comprising in its interior a second solid or liquid composition. In US672721, also provided are processes for making such article. In US6727215, alternatively, the second pouch is preferably joined to the first pouch side-by-side. In US6727215, preferred articles comprise fabric cleaning compositions for laundry, dish washing or hard surface cleaning or fabric care compositions.

US20230149267 discloses an industrial process of fabricating a readily dissolvable cartridge complex is described; a respective industrial manufacture system and a cartridge complex are further described; the process comprises: preparing an effervescent material, forming at least one segment of a segmental scaffolding, preparing a payload substance, forming structured payload substance pellets, accommodating the structured payload substance pellets in the compartments of the segmental scaffolding, compressing segment of the segmental scaffolding, iteratively forming a plurality of segments of the readily dissolvable cartridge complex.

EP4048720 discloses dissolvable solid articles comprising multiple layers of flexible, dissolvable, porous sheets, in which a coating composition is present on at least one internal surface of at least one sheet in said solid articles. The present invention also provides a process for making such solid articles.

Consumer chemicals products industry, producing liquid soaps, body wash, hair care products and many other cosmetics is one of the most polluting. One unit of a liquid soap or shampoo product typically contains: a highly pollutive disposable plastic bottle, about 20 percent of active ingredients, some of which are environmental and health-harming and about 80 percent water. Each unit of a liquid soap or shampoo product typically contains about 80 percent or more of water. Mixing and diluting hygiene product, with such a high percentage of water, enlarges their volume and causes massive pollution through their production and logistics as follows: mixing high amounts of water requires massive energy and machinery inputs in the production process for the mixing machines and conveying machinery, bottle filling machines, enormous storage spaces requirements, and internal logistics (forklifts, cranes, containers, shelves, etc.). Further to the direct environmentally harmful process, there are many highly pollutive processes involved with production and operation of equipment and machinery. Almost all the efforts and inputs, involved with the product transportation, are actually devoted to transportation of water. The water handling effect includes a direct pollutive process is grossly inefficient transport of water for thousands of miles each time by highly pollutive vessels such as ships, trains, and trucks along with all collateral logistic equipment and containers.

### SUMMARY OF THE INVENTION

**Technical Problem** The underlying technical problem of the invention is concerned with possible interactions between at least two different materials and/or substances to be dissolved in a given amount of a common solvent, to form a useful homogenous mix or solution. The presence of the first material in the solvent, dissolved at least in some effective concentration in the common solvent, can interact with and consequently affect the process of dissolution of the second material in the solution, having the first material dissolved therein.

The interaction between the first and second materials in the common solvent and impact effect of the first material, dissolved in the solvent, on the process of dissolution and/or mixing of the second material in the solution, having the first material dissolved therein, can be of a different nature, e.g. chemical, physical, etc. For instance, the first material in a non-limiting manner optionally affects the: pH, temperature, viscosity, foaming, hardness (i.e. the amount of dissolved calcium and magnesium ions), emulsifiability and/or any other parameter of the solvent, whether chemical, physical or any other. Such an impact effect, resulting from the interaction between the first and second materials in the common solvent, may adversely act on the formation of the target homogenous mix or solution.

**Solution to Problem** The effect of the interim solution, of first material dissolved in the solvent, on the process of dissolution and/or mixing of the second material therein, is initially determined. Alternatively or additionally the effect of the interim solution, of second material dissolved in the solvent, on the process of dissolution and/or mixing of the first material therein is determined. The determination is optionally performed on a theoretical and/or experimental basis. After determining the effect of the interim solution, (a) of first material dissolved in the solvent, and/or (b) of second material dissolved in the solvent, on the process of dissolution and/or mixing, respectively, of the (a) second material and/or of the (b) first material therein, a desired target effect on the overall dissolvability of a multilayered capsule in the solvent and/or on the formation of a useful homogenous mix or solution is defined.

Finally upon defining the aforementioned target effect on the overall dissolvability of a multilayered capsule in the solvent and/or on the formation of a useful homogenous mix or solution: the first material is incorporated either within the inner layer during the fabrication of the multilayered capsule or within the outer layer thereof, based on the determination of the aforementioned impact effect of the interim solution, (a) of first material dissolved in the solvent, and/or (b) of second material dissolved in the solvent, on the process of dissolution and/or mixing, respectively, of the (a) second material and/or of the (b) first material therein, as well as on the desired target effect on the overall dissolvability of a multilayered capsule in the solvent and/or on the formation of a useful homogenous mix or solution. The second material is incorporated during the fabrication, respectively, either within the outer layer of the multilayered capsule or within the inner layer thereof, based on the aforementioned determination of the impact effect of the interim solution and the desired target effect.

According to some embodiments and aspects of the present invention, there is provided a method of manufacturing a dissolvable multilayered capsule, useful for effective preparation of a cosmetic product, with a controllable arrangement of layers, having an impact effect on the dissolvability and/or mixing thereof, based on a predetermined interaction between different payload substances incorporated in different layers of the capsule, to be dissolved in a common solvent, includes the steps of: determining an impact effect of an interim solution having a given concentration of at least one first payload substance dissolved in a solvent, on a process of effective dissolution and/or mixing of at least one second payload substance in the interim solution, having the given concentration of the at least one first payload substance dissolved in the solvent; in which the at least one first payload substance is distinctively different from the at least one second payload substance; defining a desired target effect on an effective dissolution and/or mixing of the multilayered capsule in the solvent; setting a prioritizing preference for the dissolution and/or mixing of the second payload substance in the interim solution, substantially after the first payload substance was dissolved in the solvent, over a dissolution and/or mixing of the first payload substance in an alternative interim solution, having the second payload substance dissolved in the solvent, based on the determining of the impact effect, and the defining of the desired target effect on the effective dissolution and/or mixing of the multilayered capsule in the solvent; fabricating the multilayered capsule including: forming at least one discreet inner layer of the multilayered capsule; forming at least one discreet outer layer of the multilayered capsule; in which the at least one discreet inner layer of the multilayered capsule is at least partially covered by the at least one discreet outer layer of the multilayered capsule; in which the at least one first payload substance is incorporated either within the discreet inner layer or within the discreet outer layer, based on the prioritizing preference.

In some embodiments, the method further includes determining an impact effect of the alternative interim solution, having a given concentration of the at least one second payload substance dissolved in the solvent, on effective dissolution rate and/or mixing of the at least one first payload substance in the alternative interim solution, having the given concentration of the at least one second payload substance dissolved in the solvent.

In some embodiments, the target effect is selected from the group consisting of: reducing a dissolvability rate of the multilayered capsule in the solvent, thereby prolonging a dissolution time of the multilayered capsule in the solution, and increasing a dissolvability rate of the multilayered capsule in the solvent, thereby shortening the dissolution time of the multilayered capsule in the solvent.

According to some embodiments and aspects of the present invention, there is provided a dissolvable multilayered capsule, useful for effective preparation of a cosmetic product, with a controllable arrangement of layers, having an impact effect on the dissolvability and/or mixing thereof, based on a predetermined interaction between different payload substances incorporated in different layers of the capsule, to be dissolved in a common solvent, includes: at least one first payload substance to be dissolved in a solvent, in which an impact effect of an interim solution, having a given concentration of the at least one first payload substance dissolved in the solvent, on an effective dissolution rate and/or mixing of at least one second payload substance in the interim solution, having the given concentration of the at least one first payload substance dissolved in the solvent, is predetermined; at least one second payload substance to be dissolved in the interim solution; in which the at least one first payload substance is distinctively different from the at least one second payload substance; at least one discreet inner layer of the multilayered capsule; at least one discreet outer layer of the multilayered capsule; in which the at least one discreet inner layer of the multilayered capsule is essentially covered by the at least one discreet outer layer of the multilayered capsule; in which a desired target effect on an effective dissolution and/or mixing of the multilayered capsule in the solvent is predefined; in which a prioritizing preference for the dissolution and/or mixing of the second payload substance in the interim solution, substantially after the first payload substance was dissolved in the solvent, over a dissolution and/or mixing of the first payload substance in an alternative interim solution, having the second payload substance dissolved in the solvent, is preset, based on the determining of the impact effect, and the defining of the desired target effect on the effective dissolution and/or mixing of the multilayered capsule in the solvent; in which the at least one first payload substance is incorporated either within the discreet inner layer or within the discreet outer layer, based on the preset prioritizing preference.

In some embodiments, the impact effect of the alternative interim solution, having a given concentration of the at least one second payload substance dissolved in the solvent, on effective dissolution rate and/or mixing of the at least one first payload substance in the alternative interim solution, having the given concentration of the at least one second payload substance dissolved in the solvent, is further predetermined.

According to some embodiments and aspects of the present invention, there is provided a system for manufacturing a dissolvable multilayered capsule, useful for effective preparation of a cosmetic product, with a controllable arrangement of layers, having an impact effect on the dissolvability and/or mixing thereof, based on a predetermined interaction between different payload substances incorporated in different layers of the capsule, to be dissolved in a common solvent, includes: a container with at least one first payload substance to be dissolved in a solvent, in which an impact effect of an interim solution, having a given concentration of the at least one first payload substance dissolved in the solvent, on an effective dissolution rate and/or mixing of at least one second payload substance in the interim solution, having the given concentration of the at least one first payload substance dissolved in the solvent, is predetermined; a container with at least one second payload substance to be dissolved in the interim; in which the at least one first payload substance is distinctively different from the at least one second payload substance; in which a desired target effect on an effective dissolution and/or mixing of the multilayered capsule in the solvent is predefined; in which a prioritizing preference for the dissolution and/or mixing of the second payload substance in the interim solution, substantially after the first payload substance was dissolved in the solvent, over a dissolution and/or mixing of the first payload substance in an alternative interim solution, having the second payload substance dissolved in the solvent, is preset, based on the determining of the impact effect, and the defining of the desired target effect on the effective dissolution and/or mixing of the multilayered capsule in the solvent; a first feeding hopper, operationally connected to the container with the at least one first payload substance; a second feeding hopper, operationally connected to the container with the at least one second payload substance; a fabricating module including: at least one die part, including a hollow section form, essentially matching an exterior contour of the multilayered capsule, configured to form a sequential operational connection, in turn, with the first feeding hopper and with the second feeding hopper; at least one movable punch part, including an elongated form, essentially matching an interior of the hollow section form of the at least one die part, configured to exsert a pressing force onto a payload substance in the hollow section form of the at least one die part; a controller configured to form the sequential operational connection of the first feeding hopper, and in turn of the second feeding hopper, with the hollow section form of the at least one die part, in a defined order, in which the at least one first payload substance is incorporated either within the discreet inner layer or within the discreet outer layer, based on the preset prioritizing preference.

According to some embodiments and aspects of the present invention, there is provided a method of manufacturing a dissolvable multilayered capsule, useful for effective preparation of a cosmetic product, with a controllable arrangement of layers, having an impact effect on the dissolvability and/or mixing thereof, based on a predetermined interaction between different payload substances incorporated in different layers of the capsule, to be dissolved in a common solvent, includes the steps of: providing at least one first payload substance to be dissolved in a solvent; providing at least one second payload substance to be dissolved in the solvent; in which the at least one first payload substance is distinctively different from the at least one second payload substance; determining an impact effect of an interim solution having a given concentration of at least one first payload substance dissolved in the solvent, on a process of effective dissolution and/or mixing the of at least one second payload substance in the interim solution, having the given concentration of the at least one first payload substance dissolved in the solvent; defining a desired target effect on an effective dissolution and/or mixing of the multilayered capsule in the solvent; setting a prioritizing preference for dissolving the at least one first payload substance prior to the at least one second payload substance in the solvent, based on the determining of the impact effect, and the defining of the desired target effect on the effective dissolution and/or mixing of the multilayered capsule in the solvent; fabricating a multilayered capsule including: forming at least one discreet inner layer of the multilayered capsule; forming at least one discreet outer layer of the multilayered capsule; in which the at least one discreet inner layer of the multilayered capsule is essentially covered by the at least one discreet outer layer of the multilayered capsule; in which the at least one first payload substance is incorporated either within the discreet inner layer or within the discreet outer layer, based on the prioritizing preference.

According to some embodiments and aspects of the present invention, there is provided a readily dissolvable multilayered capsule with controllable arrangement of layers, expediting the dissolvability rate thereof. According to some other embodiments and aspects of the present invention, there is provided a readily dissolvable multilayered capsule with controllable arrangement of layers, prolonging and/or extending the dissolvability rate thereof. According to yet some other to be dissolved in a solvent er embodiments and aspects of the present invention, there is provided a readily dissolvable multilayered capsule with controllable arrangement of layers, with a controllable order and/or timing of the dissolvability of the layers thereof.

According to some embodiments and aspects of the present invention, there is provided a method of manufacturing a dissolvable multilayered capsule, with a predefined arrangement of layers, for controllable order and/or timing and/or duration of dissolvability thereof, including the steps of: identifying at least one first payload substance to be dissolved in a target solution; identifying at least one second payload substance to be dissolved in the target solution; in which the at least one first payload substance is distinctively different from the at least one second payload substance; predefining an order of dissolvability of the at least one first payload substance in the target solution and the at least one second payload substance in the target solution; fabricating a multilayered capsule comprising: forming at least one discreet inner layer of the multilayered capsule; forming at least one discreet outer layer of the multilayered capsule; in which the at least one discreet inner layer of the multilayered capsule is essentially covered by the at least one discreet outer layer of the multilayered capsule; incorporating the at least one payload substance, being a first in the order predefined at the step of predefining, within the inner layer of the multilayered capsule; incorporating the at least one payload substance, being a second in the order predefined at the step of predefining, within the outer layer of the multilayered capsule.

According to some other embodiments and aspects of the present invention, there are provided industrial systems for fabricating a readily dissolvable multilayered capsule with controllable arrangement of layers expediting the dissolvability rate thereof. According to yet some other embodiments and aspects of the present invention, there are provided industrial processes of fabricating a readily dissolvable multilayered capsule with controllable arrangement of layers expediting the dissolvability rate thereof.

In accordance with some aspects and embodiments of the present invention, there is provided a method of manufacturing a dissolvable multilayered capsule, with a controllable arrangement of layers expediting a dissolvability rate thereof, that includes the steps of: identifying at least one first payload substance to be dissolved in a target solution; identifying at least one second payload substance to be dissolved in the target solution; in which the at least one first payload substance is distinctively different from the at least one second payload substance; determining an effect of a given concentration of the at least one second payload substance in the target solution, on a dissolvability rate of the at least one first payload substance in the target solution, having the given concentration of the at least one second payload substance therein; determining an effect of a given concentration of the at least one first payload substance in the target solution, on a dissolvability rate of the at least one second payload substance in the target solution, having the given concentration of the at least one first payload substance therein; fabricating a multilayered capsule including: forming at least one discreet inner layer of the multilayered capsule; forming at least one discreet outer layer of the multilayered capsule; in which the at least one discreet inner layer of the multilayered capsule is essentially covered by the at least one discreet outer layer of the multilayered capsule; incorporating the at least one payload substance, having the effect determined hereinabove of a respectively higher value, within the inner layer of the multilayered capsule; incorporating the at least one payload substance, having the effect determined hereinabove of a respectively lower value, within the outer layer of the multilayered capsule.

In some embodiments, the method further includes furnishing the dissolvable multilayered capsule with a coating configured to absorb and/or neutralize undesired additives or impurities in water.

In some embodiments, the method further includes furnishing the dissolvable multilayered capsule with a coating including water softening agent, including natrium chloride.

In some embodiments, the at least one first and second payload substance comprise a surfactant.

In some embodiments, the surfactant is at least one of: sodium cocoyl glutamate, cocamidopropyl betaine, sodium cocoyl glutamate, cocamidopropyl betaine, Disodium Lauryl Sulfosuccimate, decyl glucoside, coco betaine, lauryl glucoside, sodium methyl cocoyl taurate, decyl glucoside, coco glucoside, sodium methyl oleoyl taurate, Disodium Coco-glucoside Citrate, and mixtures thereof.

In accordance with some aspects and embodiments of the present invention, there is provided a dissolvable multilayered capsule, with a controllable arrangement of layers expediting a dissolvability rate thereof, that includes: at least one first payload substance to be dissolved in a target solution; at least one second payload substance to be dissolved in the target solution; in which the at least one first payload substance is distinctively different from the at least one second payload substance; at least one discreet inner layer of the multilayered capsule; at least one discreet outer layer of the multilayered capsule; in which the at least one discreet inner layer of the multilayered capsule is essentially covered by the at least one discreet outer layer of the multilayered capsule; in which the at least one payload substance, having a respectively higher value of an effect of a given concentration of the at least one second payload substance in a target solution, on a dissolvability rate of the at least one first payload substance in the target solution, with the given concentration of the at least one second payload substance therein, incorporated within the inner layer of the multilayered capsule; in which the at least one payload substance, having a respectively lower value of an effect of a given concentration of the at least one first payload substance in a target solution, on a dissolvability rate of the at least one second payload substance in the target solution, with the given concentration of the at least one first payload substance therein, incorporated within the outer layer of the multilayered capsule.

In some embodiments, the dissolvable multilayered capsule further includes a coating configured to absorb and/or neutralize undesired additives or impurities in water.

In some embodiments, the dissolvable multilayered capsule further includes a coating including a water softening agent.

In some embodiments, the dissolvable multilayered capsule further includes a coating including natrium chloride.

In accordance with some aspects and embodiments of the present invention, there is provided a system for manufacturing a dissolvable multilayered capsule, with a controllable arrangement of layers expediting a dissolvability rate thereof, includes: a container with at least one first payload substance to be dissolved in a target solution; a container with at least one second payload substance to be dissolved in the target solution; in which the at least one first payload substance is distinctively different from the at least one second payload substance; in which an effect of a given concentration of the at least one second payload substance in the target solution, on a dissolvability rate of the at least one first payload substance in the target solution, having the given concentration of the at least one second payload substance therein, is determined; in which an effect of a given concentration of the at least one first payload substance in the target solution, on a dissolvability rate of the at least one second payload substance in the target solution, having the given concentration of the at least one second payload substance therein, is determined; a first feeding hopper, operationally connected to the container with the at least one first payload substance, a second feeding hopper, operationally connected to the container with the at least one second payload substance, a fabricating module including: at least one die part, including a hollow section form, essentially matching an exterior contour of the multilayered capsule, configured to form a sequential operational connection, in turn, with the first feeding hopper and with the second feeding hopper; at least one movable punch part, including an elongated form, essentially matching an interior of the hollow section form of the at least one die part, configured to exsert a pressing force onto a payload substance in the hollow section form of the at least one die part; a controller configured to form the sequential operational connection of the first feeding hopper in turn the second feeding hopper, with the hollow section form of the at least one die part, in a predefined order in which: a feeding hopper with the at least one payload substance, having a respectively higher determined value of the effect of the given concentration of the at least one second payload substance in the target solution, on the dissolvability rate of the at least one first payload substance in the target solution, is activated first at the predefined order, thereby feeding into the hollow section form of the at least one die part forming the at least one payload substance which forms an inner layer of the multilayered capsule; a feeding hopper with the at least one payload substance, having a respectively lower determined value of the effect of the given concentration of the at least one first payload substance in the target solution, on a dissolvability rate of the at least one second payload substance in the target solution, is activated second at the predefined order, thereby feeding into the hollow section form of the at least one die part forming the at least one payload substance which forms an outer layer of the multilayered capsule.

In some embodiments, the system further includes a pulverizator, configured for furnishing the dissolvable multilayered capsule with a coating configured to absorb and/or neutralize undesired additives or impurities in water.

In some embodiments, the invention further comprises controlling the dissolvability rate and/or dissolvability duration of at least one payload substance in the target solution, by controllably selecting a size of the particles of at least one payload substance selected from the group consisting of: at least one first payload substance and at least one second payload substance.

In some embodiments, the invention further comprises the dissolvability rate and/or dissolvability duration of at least one payload substance in the target solution, by controllably selecting a size of the particles of at least one payload substance selected from the group consisting of: at least one first payload substance and at least one second payload substance.

### DEFINITIONS

The term dissolvability or dissolved, as referred to herein, is to be construed broadly, in a non-limiting manner as including: mixing, homogenization, dispersal, dissolution, foaming, miscibility, emulsification, sublimation, chemical reaction, colorification, viscosity modification, solidification, fluidization, gasification, bubble formation, micelle formation, aggregate formation, disintegration, agglomeration, deagglomeration, aggregation, disaggregation, texturization, etc.

The term structured as referred to herein is to be construed as including any geometrical shape, exceeding in complexity a plain linear shape or a shape embodying simple cylindrical, elliptical or polygonal contour or profile. A more complex shape, a plain linear shape or a shape embodying simple cylindrical, elliptical or polygonal contour or profile, constitutes an example of structured geometry.

The terms capsule and pellet as referred to herein are to be construed as interchangeable. More specifically, a singular instance of the product is referred to as capsule. Whereas plural instances of the product, incorporated in a readily dissolvable cartridge complex, are referred to as pellets.

The term effervescent material as referred to herein is to be construed as including any material, mix of materials and/or composition capable of effervescence upon contact with water. Effervescence, exemplified in this context, can mean the emission of gas bubbles from a liquid as a result of a chemical reaction between a soluble acid source and an alkali metal carbonate to produce carbon dioxide gas.

The term payload substance as referred to herein is to be construed as including any material, mix of materials and/or composition including an anhydrous or essentially dry concentrated form and/or concentrate of any hygiene products, such as liquid soaps, body wash, hair care products and any other cosmetics, as well as any other household and/or hygiene products. The term payload substance as referred to herein can be construed as including any active ingredient of any household and/or hygiene product.

The terms method and process as used herein are to be construed as including any sequence of steps or constituent actions, regardless a specific timeline for the performance thereof. The particular steps or constituent actions of any given method or process are not necessarily in the order they are presented in the claims, description or flowcharts in the drawings, unless the context clearly dictates otherwise. Any particular step or constituent action included in a given method or process may precede or follow any other particular step or constituent action in such method or process, unless the context clearly dictates otherwise. Any particular step or constituent action and/or a combination thereof in any method or process may be performed iteratively, before or after any other particular step or action in such method or process, unless the context clearly dictates otherwise. Moreover, some steps or constituent actions and/or a combination thereof may be combined, performed together, performed concomitantly and/or simultaneously and/or in parallel, unless the context clearly dictates otherwise. Moreover, some steps or constituent actions and/or a combination thereof in any given method or process may be skipped, omitted, spared and/or opted out, unless the context clearly dictates otherwise.

By operationally connected and operably coupled or similar terms used herein is meant connected in a specific way (e.g., in a manner allowing fluid to move and/or electric power to be transmitted) that allows the disclosed system and its various components to operate effectively in the manner described herein.

The term environmentally sustainable, as referred to herein, is to be construed as including any material that is biodegradable or comprising a naturally occurring and/or excavated and/or mined material, whether in original, natural or processed form. The term environmentally sustainable, as referred to herein, is to be equally construed as including in a non-limiting manner any method or technique facilitating a reduction in: (1) energy consumption including energy consumption, whether required for manufacture, storage and/or transportation, (2) the volume or mass of disposed materials, waste or emissions, as well as (3) toxicity or non-biodegradability of disposed materials, waste or emissions.

The term dry powder, as referred to herein, may include any substance comprising one or a plurality of constituents or ingredients with one or a plurality of (average) particulate size ranges. The term low-density dry powder means dry powders having a density of about 0.8 gram per cubic centimeter or less. In particular embodiments, the low-density powder may have a density of about 0.5 gram per cubic centimeter or less. The dry powder may be with or without cohesive or agglomeration tendencies.

The term fluid or liquid as referred to herein is to be construed as any material that deforms when a shear stress is applied. While fluid generally would refer to any liquids or gases, it may be used herein to describe fluidized solids and bulk solids and/or granulate matter that are capable of flowing or otherwise moving inside a device as a result of pressure differences and/or gravitational force. Such materials may include slurries, suspensions, pastes, powders, granular solids, particle solids, granulate matter, particulate matter, as well as any combinations thereof.

The term substantially as used herein is a broad term, and is to be given its ordinary and customary meaning to a person of ordinary skill in the art (and is not to be limited to a special or customized meaning), and refers without limitation to being largely but not necessarily entirely of that quantity or quality which is specified.

The term essentially means that the composition, method or structure may include additional ingredients, stages and or parts, but only if the additional ingredients, the stages and/or the parts do not materially alter the basic and new characteristics of the composition, method or structure claimed.

As used herein, the term essentially changes a specific meaning, meaning an interval of plus or minus ten percent (± 10 percent). For any embodiments disclosed herein, any disclosure of a particular value, in some alternative embodiments, is to be understood as disclosing an interval approximately or about equal to that particular value (i.e., ± 10 percent).

As used herein, the terms about or approximately modify a particular value, by referring to a range equal to the particular value, plus or minus twenty percent (+/-20 percent). For any of the embodiments, disclosed herein, any disclosure of a particular value, can, in various alternate embodiments, also be understood as a disclosure of a range equal to about that particular value (i.e. +/-20 percent).

As used herein, the term or is an inclusive or operator, equivalent to the term and/or, unless the context clearly dictates otherwise; whereas the term and as used herein is also the alternative operator equivalent to the term and/or, unless the context clearly dictates otherwise.

The term water shall particularly include water that is fit for consumption by a living organism and/or potable water and/or suitable for topical application. In certain embodiments the living organism is a "mammal" or "mammalian", where these terms are used broadly to describe organisms which are within the class mammalia, including the orders carnivore, rodentia and primates or humans.

### DESCRIPTION OF THE DRAWINGS

**FIG 1A** is a perspective view of a readily dissolvable cartridge complex, according to some embodiments of the present invention;
**FIG 1B** is an enlarged perspective view of the readily dissolvable cartridge complex, according to some embodiments of the present invention;
**FIG 2A** is a top view of a segment of the readily dissolvable cartridge complex, according to some embodiments of the present invention;
**FIG 2B** is a perspective view of a segment of the readily dissolvable cartridge complex, according to some embodiments of the present invention;
**FIG 3A** is an isometric view of a readily dissolvable cartridge complex, according to some embodiments of the present invention;
**FIG 3B** is a top view of a segment of the readily dissolvable cartridge complex, according to some embodiments of the present invention;
**FIG 3C** is a perspective view of a compartment of a segment of the readily dissolvable cartridge complex, according to some embodiments of the present invention;
**FIG 4A** is a perspective view of a reusable dispenser bottle into which a readily dissolvable cartridge complex is introduced, according to some embodiments of the present invention;
**FIG 4B** is an exploded view of a reusable dispenser bottle into which dissolvable cartridge complex is introduced, according to some embodiments of the present invention;
**FIG 4C** is an isometric view of plurality of cups of plurality of reusable dispenser bottles in a nested and stacked configuration, according to some embodiments of the present invention;
**FIG 4D** is a side view of a cover of a reusable dispenser bottle, according to some embodiments of the present invention;
**FIG 4E** is a top view of a cover of a reusable dispenser bottle, in a closed configuration, according to some embodiments of the present invention;
**FIG 4F** is a top view of a cover of a reusable dispenser bottle, in an open configuration, according to some embodiments of the present invention;
**FIG 5** is a block diagram of an industrial manufacture system for fabricating a readily dissolvable cartridge complex, according to some embodiments of the present invention;
**FIG 6A** is an exploded view of a form and compacting mechanism complex including a stencil of an industrial manufacture system for dry fabricating of a readily dissolvable cartridge complex, according to some embodiments of the present invention;
**FIG 6B** is a schematic view of a form and compacting mechanism complex including a stencil of an industrial manufacture system for dry fabricating of a readily dissolvable cartridge complex, according to some embodiments of the present invention, showing the stencil within the form;
**FIG 7A** is an exploded view of a form and compacting mechanism complex including a structured nozzle of an industrial manufacture system for fabricating of a readily dissolvable cartridge complex, according to some other embodiments of the present invention;
**FIG 7B** is a schematic view of a structured nozzle introduced into the form, according to some other embodiments of the present invention;
**FIG 7C** is a schematic view of the formation of one segment of the readily dissolvable cartridge complex, according to some other embodiments of the present invention;
**FIG 7D** is a schematic view of the formation of a radial partition of the readily dissolvable cartridge complex, according to some other embodiments of the present invention;
**FIG 7E** is a schematic view of the readily dissolvable cartridge complex formed within the form, according to other embodiments of the present invention;
**FIG 7F** is an exploded view of the readily dissolvable cartridge complex formed within the form and a pressing array, according to some other embodiments of the present invention;
**FIG 7G** is a schematic view of a pressing piston compacting the readily dissolvable cartridge complex within the form, according to some other embodiments of the present invention;
**FIG 7H** is a schematic view of a pressing piston ejecting the readily dissolvable cartridge complex from the form, according to some other embodiments of the present invention;
**FIG 7I** is a schematic view of a pressing piston withdrawn into the from after the readily dissolvable cartridge complex was ejected from, according to some other embodiments of the present invention;
**FIG 8** is a flowchart of an industrial process of fabricating a readily dissolvable cartridge, according to some embodiments of the present invention;
**FIG 9** is a flowchart of an industrial process of essentially dry fabricating a readily dissolvable cartridge, according to some embodiments of the present invention;
**FIG 10** is a flowchart of an industrial process of essentially dry fabricating a readily dissolvable cartridge, according to some other embodiments of the present invention;
**FIG 11** is a flowchart of a method of manufacturing a dissolvable multilayered capsule, with a controllable arrangement of layers expediting a dissolvability rate thereof, according to some preferred embodiments of the present invention;
**FIG 12A** is an isometric schematic view of a dissolvable multilayered capsule with a controllable arrangement of layers expediting a dissolvability rate thereof, having an essentially sectorial shape, according to some preferred embodiments of the present invention;
**FIG 12B** is an isometric schematic view of a dissolvable multilayered capsule with a controllable arrangement of layers expediting a dissolvability rate thereof, having an essentially cylindrical or discoid shape, according to some other preferred embodiments of the present invention;
**FIG 13** is a schematic view of a system for manufacturing a dissolvable multilayered capsule, with a controllable arrangement of layers expediting a dissolvability rate thereof, according to some preferred embodiments of the present invention.

### DETAILED DISCLOSURE OF EMBODIMENTS

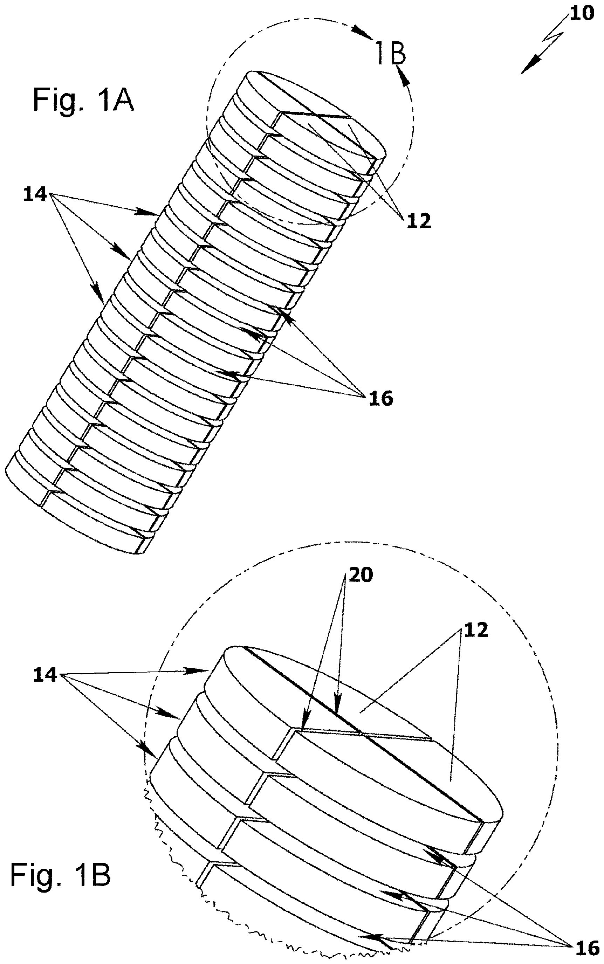

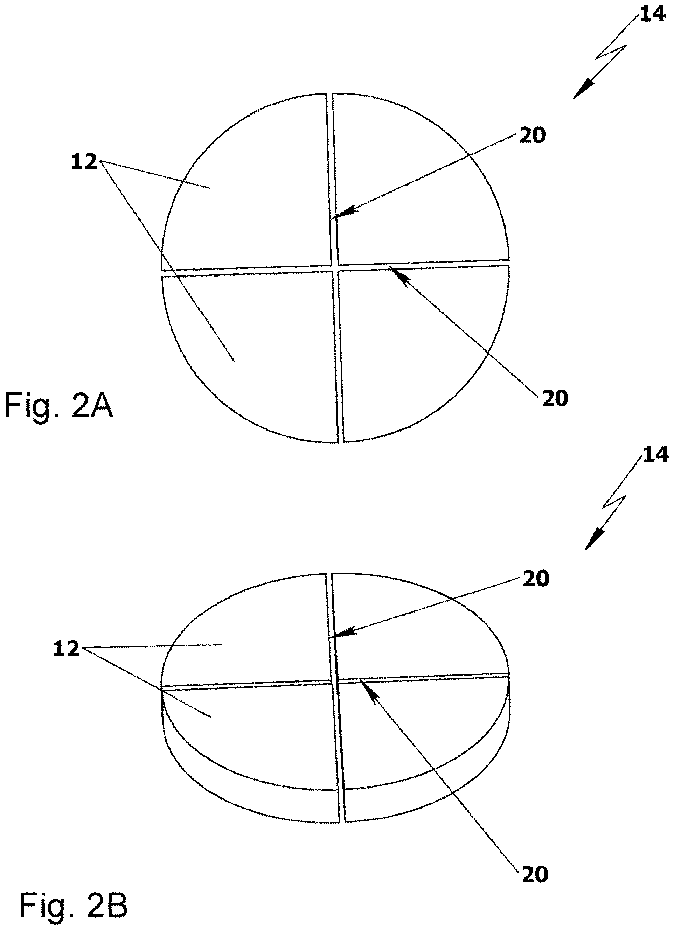

In accordance with some embodiments of the present invention, reference is now made to **FIG 1A** to **3C****,** showing readily dissolvable cartridge complex **10.** In some embodiments, readily dissolvable cartridge complex **10** comprises a plurality of structured payload substance pellets **12,** embedded within individual segments **14** of a segmental scaffolding. Segments **14** of segmental scaffolding comprises an effervescent material readily dissolvable in an aqueous solution.

The effervescent material of the segmental scaffolding may be selected from appropriate material which exhibits effervesce on contact with water. In one example, the effervescent material comprises at least sodium bicarbonate and citric acid. These materials are present in any suitable amounts to achieve effervescence. One skilled in the art is able to combine these materials to provide the desired rate of effervescence.

In other examples, the effervescent material comprises sodium bicarbonate in an amount of 40 to 75 wt percent. In other examples, the effervescent material comprises sodium bicarbonate in an amount of 50 to 70 wt percent. In other examples, the effervescent material comprises sodium bicarbonate in an amount of 55 to 70 wt percent. In some examples, the effervescent material comprises citric acid in an amount of 24 to 40 wt percent. In other examples, the effervescent material comprises citric acid in an amount of 26 to 38 wt percent based on the first effervescent material. In other examples, the effervescent material comprises citric acid in an amount of 28 to 36 wt percent. In other examples, the effervescent material comprises citric acid in an amount of 30 to 34 wt percent. In some examples, the effervescent material comprises sodium bicarbonate in an amount of 40 to 75 wt percent and citric acid in an amount of 24 to 40 wt percent.

In some embodiments, pellets **12** comprise a payload substance, including a surfactant in an amount of 0.5 to 5 wt percent. The surfactant of the payload substance is primarily selected from those surfactants known in the art to be suitable for contact with the skin. In one embodiment, the surfactant is selected from the group consisting of sodium laureth sulfate, cocamide diethanolamine, lauryl betaine and mixtures thereof. In one embodiment, the surfactant is sodium laureth sulfate.

In some embodiments, the surfactant of the payload substance provides the composition with the ability to achieve its required purpose. Thus for a cleanser, the surfactant creates foam and removes dirt and grease from the user's skin.

In some embodiments, payload substance pellets **12** further comprise a fragrance. Preferably the fragrance is present in an amount of 0.5 to 4 wt percent. In some embodiments, payload substance pellets **12** comprise fragrance in an amount of 1 to 4 wt percent. In some embodiments, payload substance pellets **12** comprise fragrance in an amount of 2 to 4 wt percent. In some embodiments, payload substance pellets **12** comprise fragrance in an amount of 2.5 to 3.5 wt percent.

In some embodiments, payload substance pellets **12** further comprise another material, whether soluble or insoluble, which us incorporated into the product to provide further effects, for example with regard to odor, taste, pH, texture colour or foam production and etc.

In some embodiments, readily dissolvable cartridge complex **10** further comprises a plurality of radial partitions **16** of segmental scaffolding. Radial partitions **16** are arranged in tandem, extending about a longitudinal centerline of dissolvable cartridge complex **10.** Radial partitions **16** divide adjacent segments **14** of the segmental scaffolding. In some embodiments, readily dissolvable cartridge complex **10** comprises plurality of segments **14.** Segments **14** are separated by radial partitions **16** and formed in-between radial partitions **16.**

In some embodiments, readily dissolvable cartridge complex **10** comprises plurality of axial partitions **20** of the segmental scaffolding. Axial partitions **20** extend transversally to and along the longitudinal centerline of dissolvable cartridge complex **10,** dividing each one of plurality of segments **14.**

In some embodiments, the segmental scaffolding of readily dissolvable cartridge complex **10** further comprises a plurality of compartments. The compartments are formed in-between axial partitions **20** of the segmental scaffolding, in each one of plurality of segments **14** of the segmental scaffolding. In some embodiments, readily dissolvable cartridge complex **10** comprises a plurality of structured payload pellets **12.** Structured payload pellets **12** are accommodated in the compartments of the segmental scaffolding.

In some embodiments, dissolvable cartridge complex **10** is configured for or even quote instantly providing for an extensive surface area of structured payload pellets **12,** upon contact with water. After the segmental scaffolding undergoes rapid or even quote instant effervescence, the accumulative surplus surface area of all structured payload pellets **12** accelerates the solubility and increasing mixing and molecule diffusion rate, by removing radial partitions **16** and axial partitions **20** of the segmental scaffolding.

In some embodiments, dissolvable cartridge complex **10** is coated by dissoluble coating **24.** When dissolvable cartridge complex **10** is placed in water or other essentially aqueous solution, dissoluble coating **24** or even quote dissolves in water. Then uncoated dissolvable cartridge complex **10** is exposed to water thereby initiating an effervescence reaction. Dissoluble coating **24** is configured to modify the water or other aqueous solution, so as to sequester, absorb, precipitate, balance or otherwise neutralize undesired impurities or additives in the water. In some examples, dissoluble coating **24** comprising a water softening agent, such as natrium chloride, configured to neutralize water hardening additives in the water, such as calcium and magnesium ions. In other examples, dissoluble coating **24** comprising a sequestering agent, such as EDTA or other chelate forming agent, configured to sequester metal ions in aqueous solution. In yet other examples, dissoluble coating **24** comprising a disinfectant, such as an oxidating or bleaching agent, configured to neutralize biological impurities in the water, for instance: iodine, chlorine, chloramine, chlorine and dioxide, and/or malodor treatment.

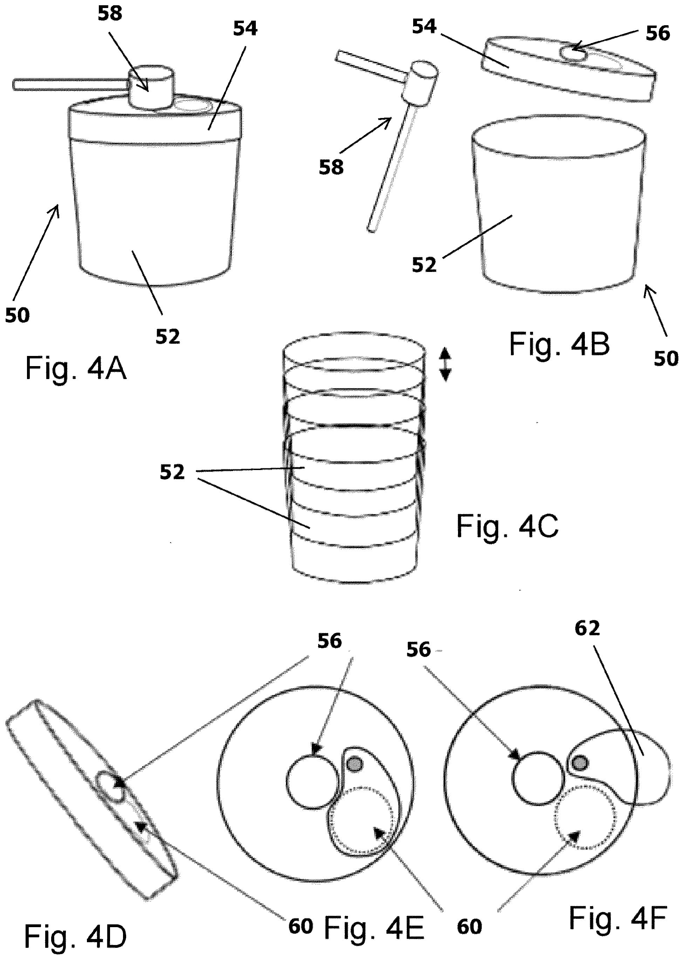

In accordance with some embodiments of the present invention, reference is now made **FIG 4A to 4F** showing reusable dispenser **50** into which dissolvable cartridge complex **10,** shown in **FIG 1A** to **3C** and described hereinabove, is administrated. The reusable dispenser of the embodiment of **FIG 4A** to **4F** illustrates various features that may be interchangeable with elements of any other embodiment described in the specification.

In some embodiments, reusable dispenser **50** comprises container **52.** Container **52** is configured for receiving the dissolvable cartridge complex, such as dissolvable cartridge complex **10** shown in **FIG 1A** to **3C****.** In some embodiments, container **52** is covered by cover **54.** Cover **54** preferably comprises aperture **56.** Aperture **56** is typically configured for accommodating dispensing pump **58.**

In some examples, dispensing pump **58** is of the type which is pressed with manual force to dispense the liquid. In other examples, dispensing pump **58** is of the electromechanical type, coupled to a power source and driven by electric power, to perform the dispensing action. In some examples, electromechanical dispensing pump **58** is manually activated upon physical contact with an electric switch, knob or button, whereas in other examples electromechanical dispensing pump **58** is configured for contactless activation, typically by an IR detector. In some examples, dispensing pump **58** comprises a foam dispenser, comprising a mechanism configured to transform the liquid dispensed from reusable dispenser **50** into a light or thick foam.

In some embodiments, cover **54** of reusable dispenser **50** further comprises cartridge aperture **60.** The dissolvable cartridge complex, such as dissolvable cartridge complex **10** shown in **FIG 1A** to **3C****,** is introduced into container **52** through cartridge aperture **60.** Cartridge aperture **60** is optionally coverable by lid **62.** Preferably, lid **62** is operationally connected to cover **54.** It should be acknowledged that the configuration of reusable dispenser **50** with cover **54,** with cover on top, with cartridge aperture **60** is merely exemplary, whereas any other configuration is equally applicable.

In some embodiments reusable dispenser bottle **50** is eco-efficient and reusable as well as recyclable. In some examples, reusable dispenser bottle **50** is made of stainless steel and/or recycled aluminum and/or any other material or composition. In other embodiments, reusable dispenser bottle **50** is disassembled so that a plurality of containers **52** and plurality of covers **54** are nested in a stack one on top of another, such as shown in **FIG 4C****,** thereby reducing shipping volume of dispensers **50.**

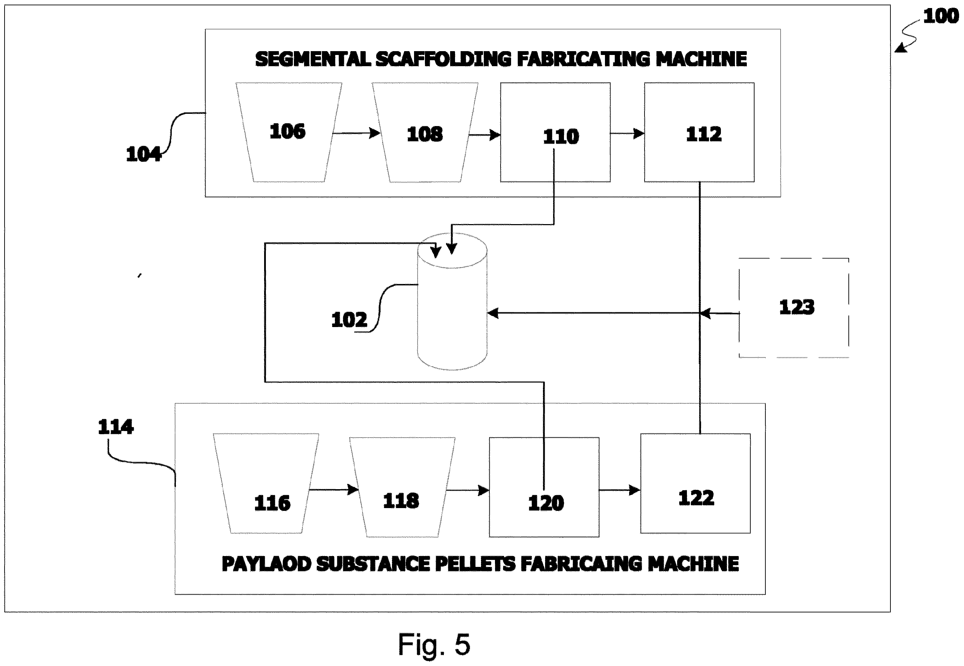

In accordance with some embodiments of the present invention, reference is now made to **FIG 5****,** showing industrial manufacture system **100** for fabricating a readily dissolvable cartridge complex. Industrial manufacture system **100** of the embodiment of **FIG 5** illustrates various features that may be interchangeable with elements of any other embodiment described in the specification.

In some embodiments, system **100** for fabricating a readily dissolvable cartridge complex comprises at least one form **102.** Form **102** comprising an essentially hollow shape, in which an interior surface of at least one form **102** essentially conforms and/or matches with a shape of dissolvable cartridge complex, such as dissolvable cartridge complex **10** shown in **FIG 1A** to **3C** and described hereinabove.

In some embodiments, system **100** further comprises segmental scaffolding fabricating machine **104.** In some embodiments, segmental scaffolding fabricating machine **104** comprises effervescent material reservoir **106.** Effervescent material reservoir **106** contains and stores an effervescent material readily dissolvable in an aqueous solution. In some examples, effervescent material reservoir **106** comprises a conical stainless steel reservoir or a miniature silo. In some embodiments, the effervescent material effervescent material in effervescent material reservoir **106** is typically in the form of dry powder. The term dry powder, as referred to herein, may include any substance comprising one or a plurality of constituents or ingredients with one or a plurality of (average) particulate size ranges.

In some embodiments, segmental scaffolding fabricating machine **104** comprises dosing module **108.** Dosing module **108** is connected to effervescent material reservoir **106** and configured to receive the effervescent material from effervescent material reservoir **106.** Dosing module **108** is configured to portion a predetermined amount of the effervescent material.

In some examples, dosing module **108** comprises a predefined volume and/or a weighting scale device. Dosing module **108** is configured to portion the effervescent material of various predetermined amounts, volumes and or weights. From many kilograms and bulk volume of effervescent material in reservoir **106,** dosing module **108** portions relatively small and predetermined amounts.

In some embodiments, segmental scaffolding fabricating machine **104** comprises fabricating module **110.** Fabricating module **110** comprises at least one nozzle, configured to dispose the effervescent material in a predefined structured arrangement within form **102,** thereby forming a plurality of compartments, within at least one segment of the segmental scaffolding. In some preferred embodiments, fabricating module **110** comprises at least one nozzle, configured to dispose the effervescent material in a predefined location and/or coordinate within form **102** in a controllable manner, somewhat similarly to a jet printer, thereby gradually forming the radial and axial portions step-by-step, of at least one segment of the segmental scaffolding.

In some embodiments, segmental scaffolding fabricating machine **104** comprises compacting mechanism **112.** Compacting mechanism **112** comprises at least one pressing piston operationally connected to pressure exerting device **123,** configured to compress at least one segment of the readily dissolvable cartridge complex within form **102.** In some embodiments, when the effervescent material is compressed, it undergoes agglomeration and is capable thereafter to inherently maintain the structural integrity of the readily dissolvable cartridge complex.

In some embodiments, system **100** is configured for semi-liquefied fabricating of the readily dissolvable cartridge complex. In such embodiments, segmental scaffolding fabricating machine **104** of system **100** further comprises a saturating module, configured for controllably saturating the effervescent material, with a predefined amount of saturating substrate, thereby conferring to the effervescent material a semi-liquefied consistency. In some examples, the saturating module comprises a substrate storage unit and/or a substrate dosimeter. The substrate storage unit is configured storing the substrate or liquid added to the effervescent material, whereas the substrate dosimeter is configured for controllably adding a certain amount of substrate or liquid to the effervescent material, thereby conferring to the effervescent material a semi-liquefied consistency.

In some embodiments, segmental scaffolding fabricating machine **104** comprises a blender. The blender is configured for mixing the dosed saturating substrate or liquid with a predefined amount of effervescent material, thereby conferring homogeneity to the effervescent material, optionally with semi-liquefied consistency. In some embodiments, system **100** further comprises structured payload substance pellets fabricating machine **114.** In some embodiments, structured payload substance pellets fabricating machine **114** comprises payload substance reservoir **116.** In some embodiments, the payload substance in payload substance reservoir **116** is typically in the form of dry powder. Payload substance reservoir **116** contains and stores a payload substance. In some examples, payload substance reservoir **116** comprises a conical stainless steel reservoir or a miniature silo.

In some embodiments, structured payload substance pellets fabricating machine **114** further comprises dosing module **118.** Dosing module **118** is connected to payload substance reservoir **116** and configured to receive the payload substance from reservoir **116.** Dosing module **118** is configured to portion a predetermined amount of the payload substance. Dosing module **108** is configured to portion a predetermined amount of the payload substance.

In some embodiments, structured payload substance pellets fabricating machine **114** comprises fabricating module **120.** In some embodiments, payload substance pellets fabricating module **120** comprises at least one nozzle, configured to dispose the payload substance, optionally having a semi-liquefied consistency. In such embodiments, the at least one nozzle is configured to dispose the payload substance in a predefined structured arrangement within form **102,** thereby forming a plurality of structured payload substance pellets within the compartments of the segmental scaffolding, in at least one segment of the of the readily dissolvable cartridge complex. In some preferred embodiments, fabricating module **120** comprises at least one nozzle, configured to dispose the payload substance in a predefined location and/or coordinate within form **102** in a controllable manner, somewhat similarly to a jet printer, thereby gradually forming the structured payload substance pellets step-by-step, of at least one segment of the readily dissolvable cartridge complex.

In some embodiments, the at least one nozzle of fabricating module **110** configured to dispose the effervescent material in a predefined location and/or coordinate within form **102,** as well as the at least one nozzle of fabricating module **120** configured to dispose the payload substance in a predefined location and/or coordinate within form **102,** in a controllable manner somewhat similarly to a jet printer, thereby gradually forming step-by-step the segment of the segmental scaffolding, are exchangeable nozzles on a same disposing machine, such as an extruder, dispenser, press, pump or ejector.

Fabricating module **110** comprises at least one nozzle, configured to dispose the effervescent material in a predefined structured arrangement within form **102,** thereby forming a plurality of compartments, within at least one segment of the segmental scaffolding. In some preferred embodiments, fabricating module **110** comprises at least one nozzle, configured to dispose the effervescent material in a predefined location and/or coordinate within form **102** in a controllable manner, somewhat similarly to a jet printer, thereby gradually forming the radial and axial portions step-by-step, of at least one segment of the segmental scaffolding.

In some preferred embodiments, fabricating module **120** comprises at least one structured nozzle, for instance embodying the shape of the segmental scaffolding and/or of the structured payload substance pellets and/or any portion thereof. A structured nozzle optionally disposes an entire fragment of at least one segment of the readily dissolvable cartridge complex, at once.

In some embodiments, structured payload substance pellets fabricating machine **114** further comprises compacting mechanism **122.** Compacting mechanism **122** comprises at least one pressing piston operationally connected to pressure exerting device **123,** configured to compress the structured payload substance pellets within the compartments of the segmental scaffolding, thereby accommodating the structured payload substance pellets in the compartments, formed within at least one segment of the segmental scaffolding of the readily dissolvable cartridge complex, within form **102.**

In some embodiments, system **100** is configured for semi-liquefied fabricating of the readily dissolvable cartridge complex. In such embodiments, structured payload substance pellets fabricating machine **114** of system **100** further comprises a saturating module, configured for controllably saturating the payload substance, with a predefined amount of saturating substrate, thereby conferring to the payload substance a semi-liquefied consistency. In some examples, the saturating module comprises a substrate storage unit and/or a substrate dosimeter. The substrate storage unit is configured storing the substrate or liquid added to the payload substance, whereas the substrate dosimeter is configured for controllably adding a certain amount of substrate or liquid to the payload substance, thereby conferring to the payload substance a semi-liquefied consistency.

In some embodiments, structured payload substance pellets fabricating machine **114** comprises a blender. The blender is configured for mixing the dosed saturating substrate or liquid with a predefined amount of payload substance, thereby conferring homogeneity to the payload substance with semi-liquefied consistency.

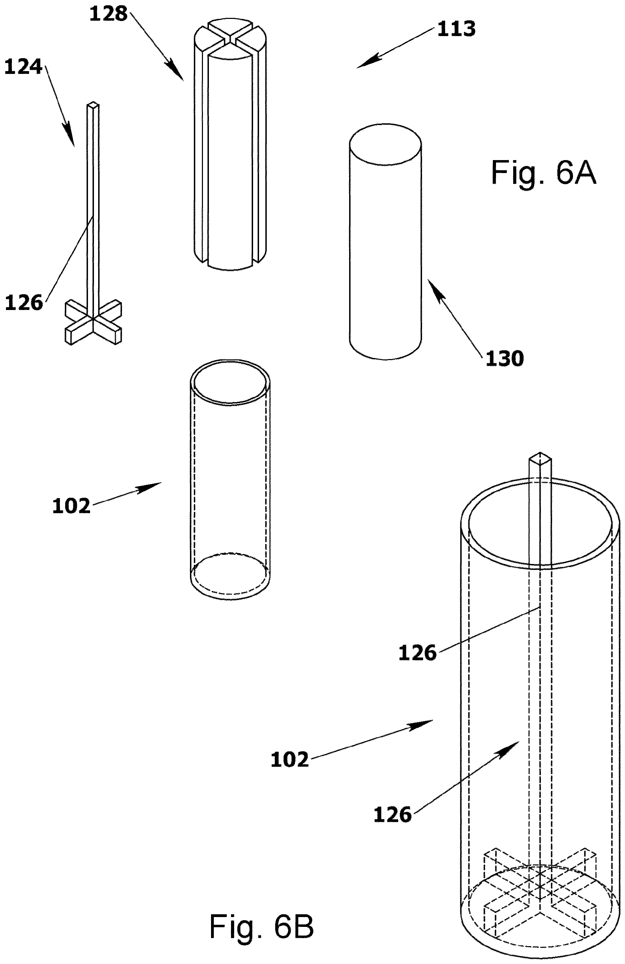

Reference is now made to **FIG 6A** and **6B****,** showing an example of assembly **113,** including the constituents of form **102,** fabricating module **110** and compacting module **112** of the segmental scaffolding fabricating machine **104** and fabricating module **120** and compacting module **122** of the structured payload substance pellets fabricating machine **114** of the industrial manufacture system **100** shown in **FIG 5****,** configured for essentially dry fabricating of a readily dissolvable cartridge complex. The example of assembly **113** of the embodiment of **FIG 6A** and **6B** illustrates various features that may be interchangeable with elements of any other embodiment described in the specification.

In some embodiments, the industrial manufacture system comprises at least one stencil **124.** In some embodiments, the exterior outline of least one stencil **124** essentially conforms and/or matches with a shape of axial partitions of the segmental scaffolding, extending transversally to and along the longitudinal centerline of the dissolvable cartridge complex. In some embodiments, at least one stencil **124** comprises mandrel **126.** Mandrel **126** is configured for manipulating the distal part of stencil **124** within form **102.**

In some embodiments, compacting mechanism **112** comprises pellet pressing piston array **128.** The exterior outline of the pistons, in pellet pressing piston **128,** essentially conforms and/or matches with exterior outline of a shape of the structured payload substance pellets. Pellet pressing piston array **128** is operationally connected to pressure exerting device **123,** configured to compress the structured payload substance pellets, within form **102.**

In some embodiment, compacting mechanism **112** further comprises segment pressing piston **130** operationally connected to pressure exerting device **123.** Segment pressing piston **130** configured to compress at least one segment of the segmental scaffolding of the readily dissolvable cartridge complex within form **102.**

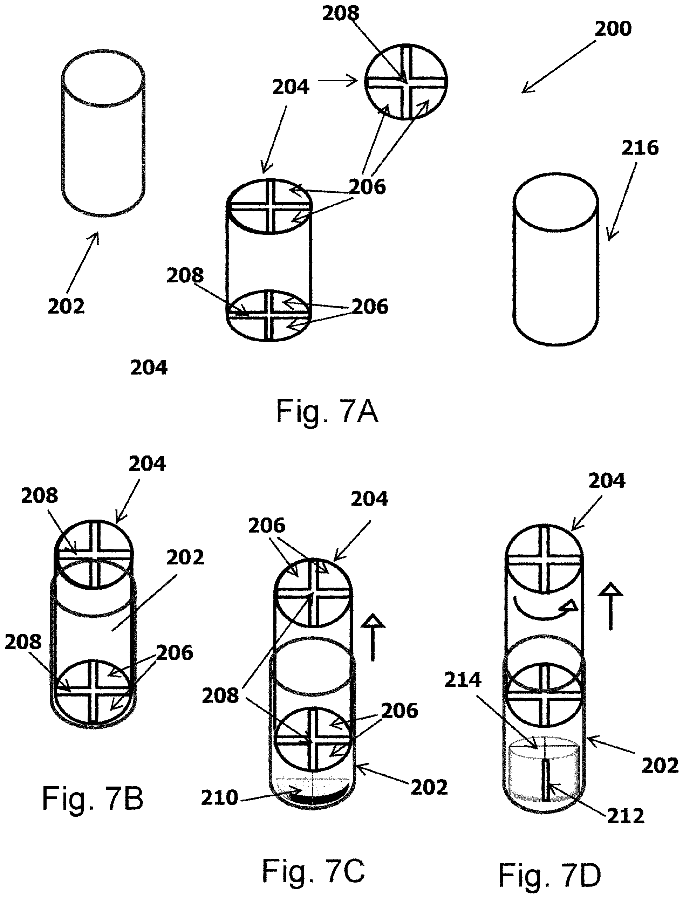

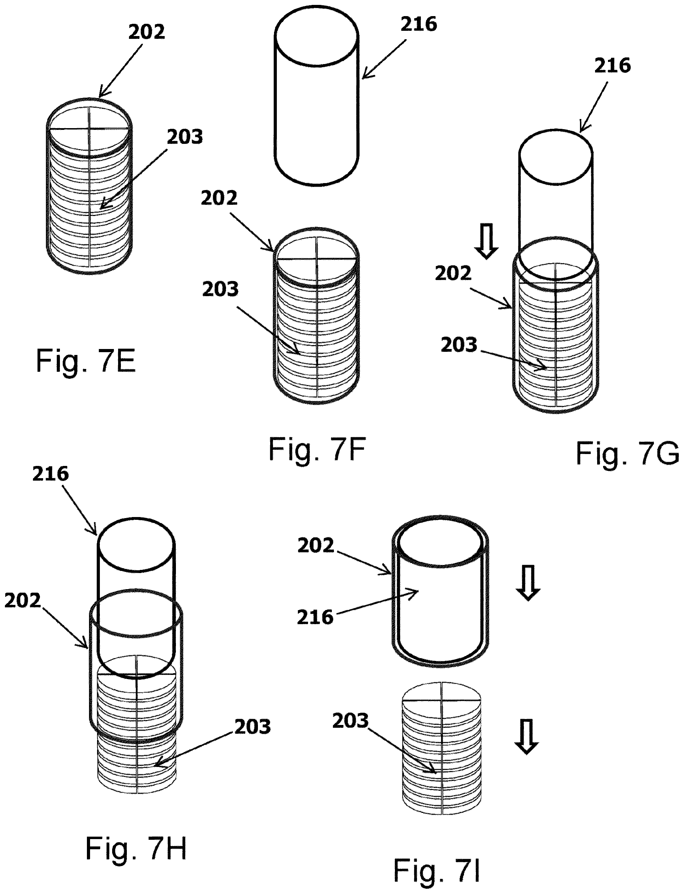

Reference is now made to **FIG 7A** to **7I****,** showing another example of assembly **200,** including the constituents of form **102,** fabricating module **110** and compacting module **112** of the segmental scaffolding fabricating machine **104** and fabricating module **120** and compacting module **122** of the structured payload substance pellets fabricating machine **114** of the industrial manufacture system **100** shown in **FIG 5****,** configured for essentially dry fabricating of a readily dissolvable cartridge complex. The example of assembly **200** of the embodiment of **FIG 7A** to **7I** illustrates various features that may be interchangeable with elements of any other embodiment described in the specification.

In some embodiments, assembly **200** comprises form **202.** Form **202** comprising an essentially hollow shape, in which an interior surface of form **202** essentially conforms and/or matches with a shape of dissolvable cartridge complex **203,** such as dissolvable cartridge complex **10** shown in **FIG 1A** to **3C** and described hereinabove. It should be appreciated that the shape of the dissolvable cartridge complex, such as complex **10** shown in **FIG 1A** to **3C** and/or complex **203,** is shown as cylindrical merely by a way of example, whereas numerous profiled shapes, such as rectangular, elliptical, quadratic, triangular, polygonal and/or structured are equally applicable.

In some embodiments, assembly **200** further comprises structured nozzle **204.** The exterior outline of structured nozzle **204** essentially conforms and/or matches with exterior outline of dissolvable cartridge complex **203,** whereas the shape of the interior outline of structured nozzle **204** essentially conforms and/or matches with exterior outline the structured payload substance pellets and with a shape of axial partitions of the segmental scaffolding, extending transversally to and along the longitudinal centerline of dissolvable cartridge complex **203.**

In some embodiments, structured nozzle **204** comprises a plurality of payload substance nozzles **206.** Payload substance nozzles **206** are configured for administrating the payload substance into form **202,** thereby forming a plurality of structured payload substance pellets. In some embodiments, structured nozzle **204** comprises effervescent material nozzle **208.** Effervescent material nozzle **208** is configured for administrating the effervescent material into form **202,** thereby forming the axial partitions between the pellets, as well as the radial partitions between the segments.

In some embodiments, structured nozzle **204** with plurality payload substance nozzles **206** and effervescent material nozzle **208** is insertable into form **202.** Payload substance nozzles **206** and effervescent material nozzle **208** are implementable for administrating, optimally concomitantly, the payload substance and the effervescent material, while vertically translating structured nozzle **204** within form **202** in an upward direction, thereby forming first segment **210** of dissolvable cartridge complex **203** within form **202.**

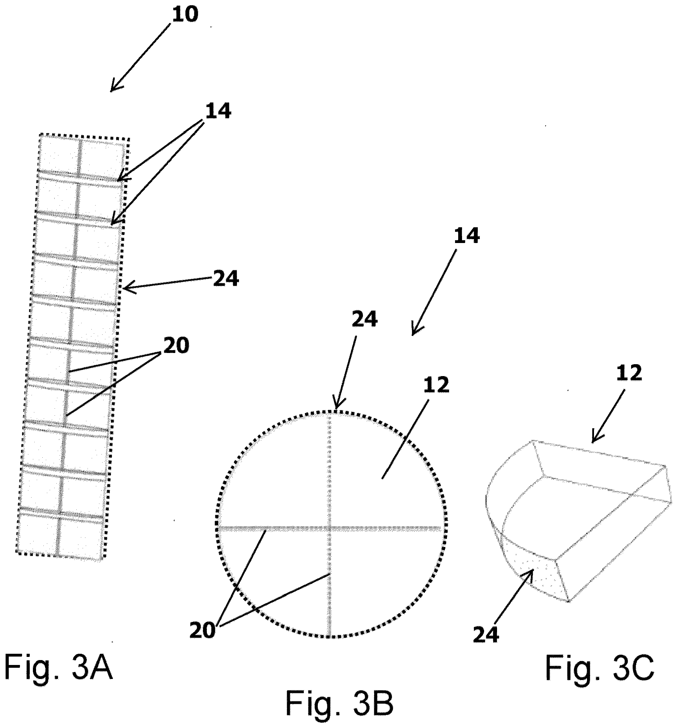

In some embodiments, structured nozzle **204** is rotatable about longitudinal centerline of structured nozzle **204** while additionally filling from effervescent material nozzle **208** a layer of effervescent material above constructed segment **210,** thereby providing material for radial partition **212,** such as radial partitions **16** shown in **FIG 3A****,** between the segments of dissolvable cartridge complex **203.**

In some embodiments, upon administering the effervescent material for radial partition **214,** payload substance nozzles **206** and effervescent material nozzle **208** are implementable for administrating, optimally concomitantly, the payload substance and the effervescent material, while vertically translating structured nozzle **204** within form **202** in an upward direction, thereby forming the next segment of dissolvable cartridge complex **203.**

In some embodiments, assembly **200** further comprises pressing piston **216.** Pressing piston **216** is insertable into form **203** once structured nozzle **204** is withdrawn therefrom. The exterior outline of pressing piston **216,** essentially conforms and/or matches with exterior outline of a shape of the readily dissolvable cartridge complex. Pressing piston **210** is configured to compact readily dissolvable cartridge complex **203** within form **202** and/or for ejecting readily dissolvable cartridge complex **203** from form **202.**

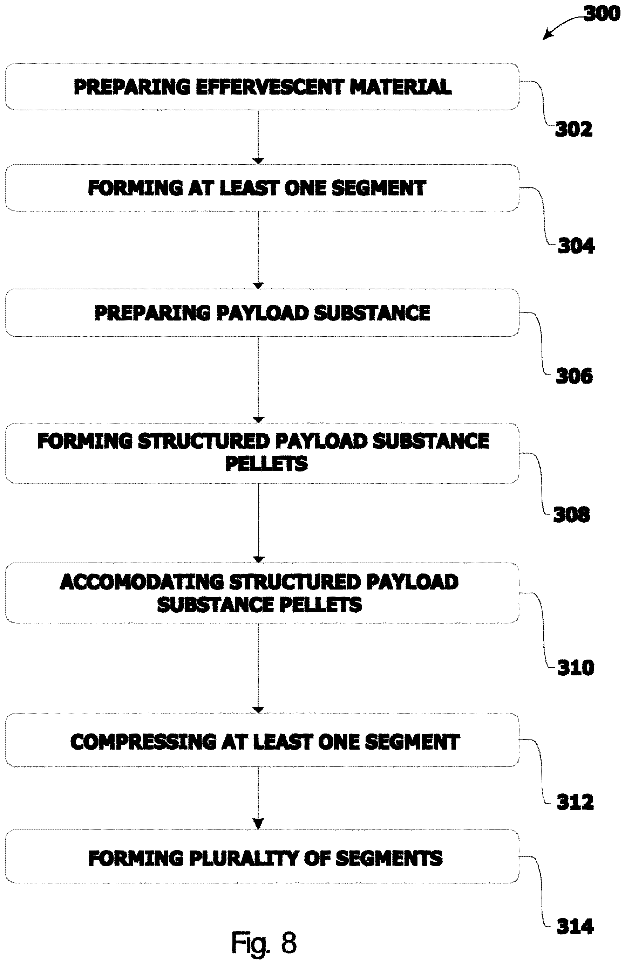

In accordance with some embodiments of the present invention, reference is now made **FIG 8** showing a flowchart of industrial process **300** of fabricating a readily dissolvable cartridge complex. The process of the embodiment of **FIG 8** illustrates various features that may be interchangeable with elements of any other embodiment described in the specification.

In some embodiments, process **300** commences with step **302** of preparing an effervescent material readily dissolvable in an aqueous solution. In some embodiments, process **300** comprises step **304** of forming at least one segment of a segmental scaffolding from the effervescent material. Step **304** typically further comprises forming at least on radial partition of at least one segment of the segmental scaffolding, extending about the longitudinal centerline of the dissolvable cartridge complex.

In some embodiments, step **304** comprises forming a plurality of axial partitions within at least one segment of the segmental scaffolding, extending transversally to and along the longitudinal centerline of the dissolvable cartridge complex. In some embodiments, step **304** further comprises forming a plurality of compartments formed in-between the axial partitions, in each one from the plurality of the segments of the segmental scaffolding.

In some embodiments, process **300** includes step **306** of preparing a payload substance. In some embodiments, process **300** further comprises step **308** of forming a plurality of structured payload substance pellets. In some preferred embodiments, step **304** of forming at least one segment of a segmental scaffolding from the effervescent material is performed concomitantly with step **308** of forming a plurality of structured payload substance pellets, by a plurality of nozzles.

In some embodiments, process **300** includes step **310** of accommodating the structured payload substance pellets in the compartments of the segmental scaffolding. In some embodiments, process **300** further comprises step **312** of compressing at least one segment of the segmental scaffolding of the readily dissolvable cartridge complex.

In some embodiments, process **300** includes a step of deploying at least one stencil, in which an exterior outline of at least one stencil essentially conforms and/or matches with a shape of axial partitions of the segmental scaffolding, extending transversally to and along the longitudinal centerline of the dissolvable cartridge complex. In some embodiments, process **300** includes step of deploying at least one stencil, in which an exterior outline of at least one stencil essentially conforms and/or matches with a shape of the structured payload substance pellets.

In some embodiments, process **300** includes a step of saturating the payload substance and the effervescent material, with a predefined amount of saturating substrate, thereby conferring the payload substance and the effervescent material a semi-liquefied consistency. The term semi-liquefied consistency comprises a slurry and/or sludge like consistency. In some embodiments, process **300** includes a step of controllably delivering the payload substance and the effervescent material, into a predetermined location within the form.

In some embodiments, process **300** comprises step **314** of iteratively forming a plurality of segments of the readily dissolvable cartridge complex separated by the radial partitions.

In some embodiments, process **300** further includes a step of furnishing the readily dissolvable cartridge complex with a coating configured to absorb and/or neutralize undesired additives or impurities in water. In some embodiments, process **300** includes further a step of furnishing the readily dissolvable cartridge complex with a coating comprising water softening agent, including natrium chloride.

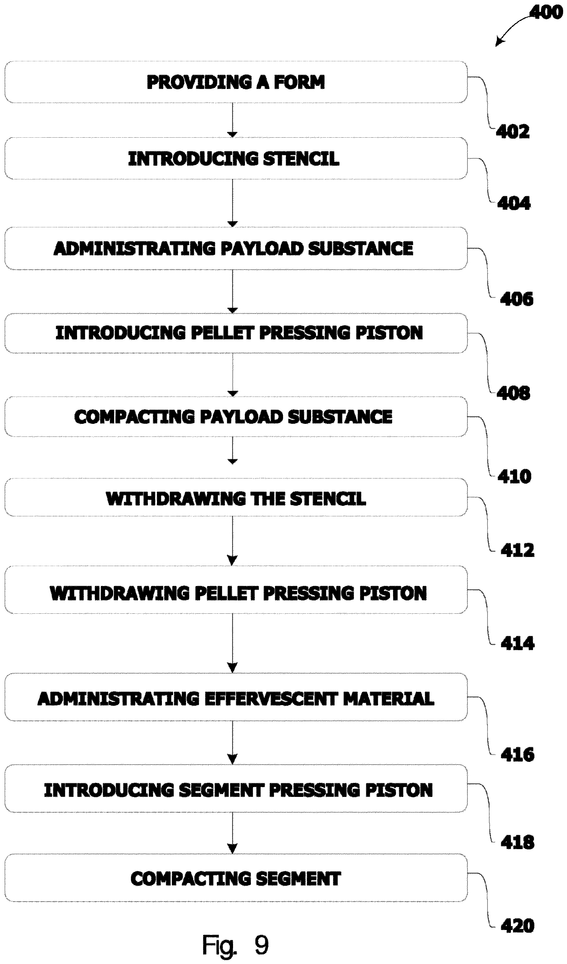

In accordance with some embodiments of the present invention, reference is now made **FIG 9** showing a flowchart of industrial process **400** of essentially dry fabricating of the readily dissolvable cartridge. The process of the embodiment of **FIG 9** illustrates various features that may be interchangeable with elements of any other embodiment described in the specification.

In some embodiments, process **400** commences with step **402** of providing a form, such as form **102** shown in **FIG 6A** and **6B****.** In some embodiments, process **400** further comprises step **404** of introducing a stencil, such as stencil **124** shown in **FIG 6A** and **6B****,** into the form.

In some embodiments, process **400** further includes step **406** of administrating the payload substance into the form. Step **406** typically comprises flattening and/or leveling the payload substance using vibration and/or a dedicated tool and/or conveying mini screws and/or air pressure and/or mechanical pressure. In some embodiments, process **400** further comprises step **408** of introducing a pellet pressing piston, such as pellet pressing piston array **128** shown in **FIG 6A** and **6B****,** into the form. The pellet pressing piston essentially the exterior outline of the structured payload substance pellet.

In some embodiments, process **400** further comprises step **410** of compacting the payload substance, thereby forming the plurality of structured payload substance pellets, within one segment. In some embodiments, step **406** of administrating the payload substance into the form as well as step **410** of compacting the payload substance are performed in combination and a *priori*, so that the plurality of structured payload substance pellets is pre-pressed ahead, whereby already compacted structured payload substance pellets are administered into the form. Process **400** In some embodiments, process **400** further includes step **412** of withdrawing the stencil from the form.

In some embodiments, process **400** further comprises step **414** of withdrawing the pellet pressing piston from the form. In some embodiments, process **400** further includes step **416** of administrating an effervescent material into the form, thereby filling the void spaces between the structured payload substance pellets thereby forming the axial partitions between the pellets, as well as additionally filling a layer above the structured payload substance pellets in the constructed segment, thereby providing material for the radial partition, such as radial partitions **16** shown in **FIG 3A****,** between the segments. In some embodiments, step **416** further comprises step of flattening and/or leveling the effervescent material using vibration and/or a dedicated tool and/or conveying mini screws and/or air pressure and/or mechanical pressure.

In some embodiments, process **400** further comprises step **418** of introducing a segment pressing piston, such as segment pressing piston **130** shown in **FIG 6A****,** into the form. The segment pressing piston assumes essentially the entire cross-sectional area of the form. In some embodiments process **400** includes step **420** of compacting, thereby forming a segment within the readily dissolvable cartridge complex. In some embodiments, process **400** yet further comprises a step of withdrawing the segment pressing piston from the form. In some embodiments, process **400** ultimately includes an iterative step of repeating steps **404** to 420.

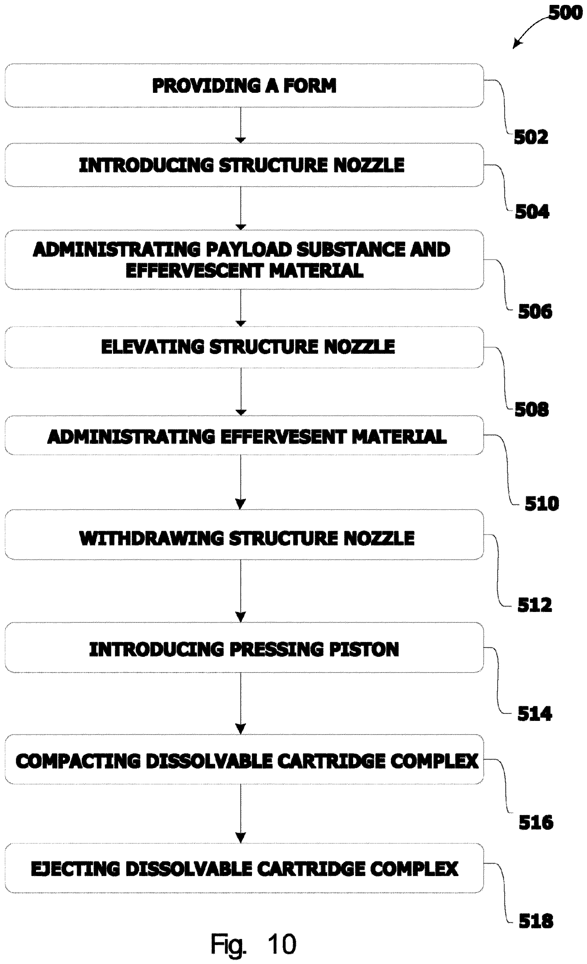

In accordance with another embodiment of the present invention, reference is now made **FIG 10** showing a flowchart of industrial process **500** of essentially dry fabricating of the readily dissolvable cartridge. The process of the embodiment of **FIG 10** illustrates various features that may be interchangeable with elements of any other embodiment described in the specification.

In some embodiments, process **500** commences with step **502** of providing a form, such as form **202** shown in **FIG 7A** to **7I****.** In some embodiments, process **500** further comprises step **504** of introducing a structured nozzle, such as structured nozzle **204** shown in **FIG 7A** to **7I****,** into the form. The structured nozzle comprises a plurality of dedicated payload substance nozzles and an effervescent material nozzle.

In some embodiments, process 500 further includes step 506 of administrating, optionally simultaneously, the payload substance and the effervescent material into the form, via the structured nozzle. Step **506** typically comprises flattening and/or leveling the payload substance using vibration and/or a dedicated tool and/or conveying mini screws and/or air pressure and/or mechanical pressure. In some embodiments, process **500** further comprises step **508** of elevating the structured nozzle by axial translation of the structured nozzle within the form in an upward direction, thereby forming a segment of the readily dissolvable cartridge complex.

In some embodiments, process **500** further comprises step **510** of administrating a layer of effervescent material, optionally from the effervescent material nozzle, while elevating by axial translation of the structured nozzle within the form in an upward direction and optionally rotating the structured nozzle, thereby providing material for the radial partition, such as radial partition **16** shown in **FIG 3A****,** between the segments. Step **510** typically comprises flattening and/or leveling the effervescent material. In some embodiments, process **500** ultimately includes an iterative step of repeating steps **506** to **510.**

In some embodiments, process **500** further comprises step **512** of withdrawing the structured nozzle from the form once the entire readily dissolvable cartridge complex and/or a single segment of the readily dissolvable cartridge complex is formed. In some embodiments, process **500** further comprises step **514** of introducing a pressing piston, such as segment pressing piston **130** shown in **FIG 6A** and **6B****,** into the form. The segment pressing piston typically assumes essentially the entire cross-sectional area of the form.

In some embodiments, process **500** further comprises step **516** of compacting the readily dissolvable cartridge complex. In some embodiments, process **500** further concluded with step **518** of ejecting the readily dissolvable cartridge complex from the form.

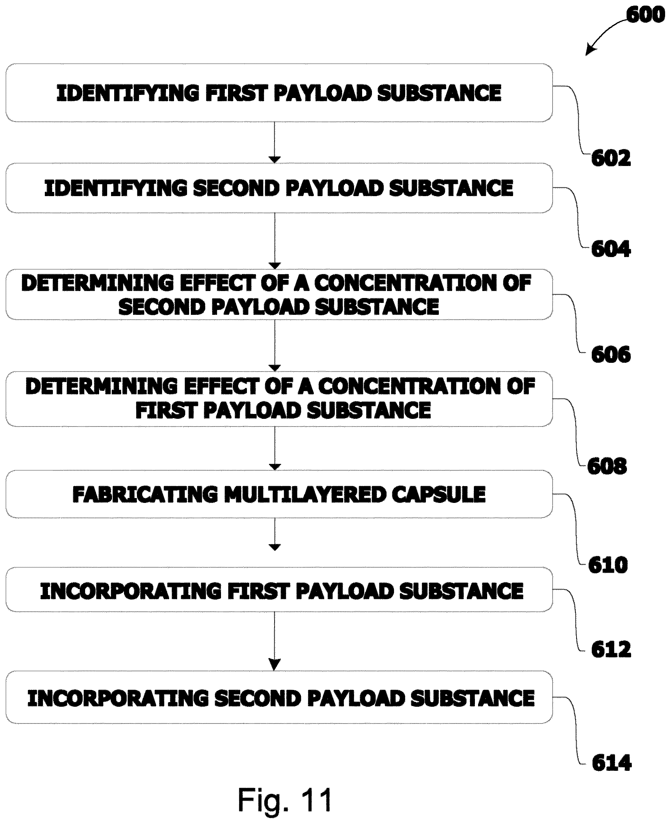

In accordance with some embodiments of the present invention, reference is now made **FIG 11** showing the flowchart of method **600** of manufacturing a dissolvable multilayered capsule, with a controllable arrangement of layers, expediting a dissolvability rate thereof. In some embodiments, method **600** comprises step **602** of identifying at least one first payload substance to be dissolved in a target solution. Target solution, also referred herein as a solvent solution, is typically an aqueous solution or water, as defined hereinabove.

In some embodiments, method **600** further comprises step **604** of identifying at least one second payload substance to be dissolved in the target solution. In some embodiments, at least one first payload substance is distinctively different from at least one second payload substance.

In some embodiments, method **600** further comprises step **606** of determining an effect of a given concentration of at least one second payload substance in the target solution, on a dissolvability rate of at least one first payload substance in the target solution, having the given concentration of at least one second payload substance therein.

In some embodiments, method **600** further includes step **608** of determining an effect of a given concentration of at least one first payload substance in the target solution, on a dissolvability rate of at least one second payload substance in the target solution, having the given concentration of at least one first payload substance therein.

In some embodiments, method **600** further comprises a step of controlling the dissolvability rate and/or dissolvability duration of at least one first payload substance and at least one second payload substance in the target solution, by controlling the density of the particles and/or the particles size of at least one first payload substance and/or at least one second payload substance. In some embodiments, exerting greater or lesser pressure force onto at least one payload substance results in greater or lesser density of the layer it forms, which respectively effects the dissolution rate of the given layer in the multilayered capsule.

In some examples, method **600** further includes a step of controlling the density of at least one payload substance by adding a disintegrant material to at least one of the first and/or second payload substances. Adding at least one disintegrant material facilitates the minimization of the pression force exerted onto at least one payload substance, thereby minimizing the density of the respective layer and accelerating the dissolution rate of the respective layer of the multilayered capsule. In some examples, the disintegrant material is selected of: starch, pregelatinized starch, solka floc, kaolin, bentonite, alginic acid, veegum, and mixtures thereof.

In some examples, method **600** further comprises a step of controlling the density of at least one payload substance by adding a binder material to at least one of the first and/or second payload substances. In some examples, the binder material is selected of: pregelatinized starch, synthetic polymers, and mixtures thereof.

In some embodiments, method **600** further includes step **610** of fabricating a multilayered capsule. Step **610** comprises forming at least one discreet inner layer of the multilayered capsule. In some embodiments, step **610** further includes a step of forming at least one discreet outer layer of the multilayered capsule. In some embodiments, at least one discreet inner layer of the multilayered capsule is essentially covered by at least one discreet outer layer of the multilayered capsule.

In some embodiments, method **600** further includes step **612** of incorporating at least one payload substance, having the effect determined at steps **606** and **608** of a respectively higher value, within the inner layer of the multilayered capsule.

In some embodiments, method **600** further comprises step **614** of incorporating at least one payload substance, having the effect determined at steps **606** and **608** of a respectively lower value, within the outer layer of the multilayered capsule.

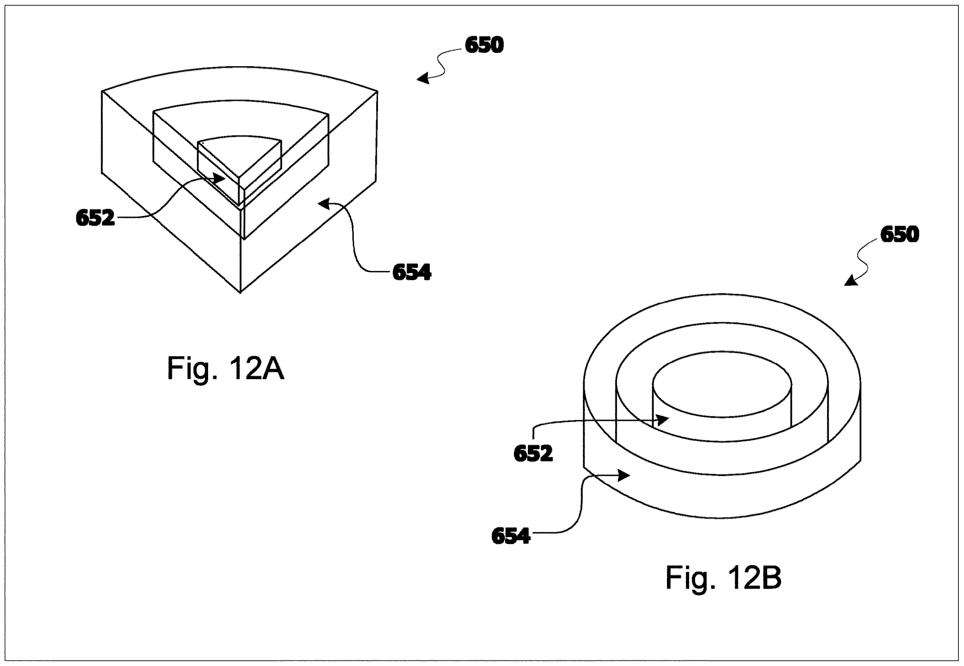

In accordance with some embodiments of the present invention, reference is now made **FIG 12A** and **12B****,** showing dissolvable multilayered capsules **650** of different shapes, with a controllable arrangement of layers expediting a dissolvability rate thereof. The dissolvable multilayered capsule of the embodiment of **FIG 12A** and **12B** illustrates various features that may be interchangeable with elements of any other embodiment described in the specification. In some embodiments, dissolvable multilayered capsule **650** comprises at least one first payload substance to be dissolved in a target solution. In some embodiments, dissolvable multilayered capsule **650** further comprises at least one second payload substance to be dissolved in the target solution. In some embodiments, at least one first payload substance is distinctively different from at least one second payload substance.

In some embodiments, at least one of the first and second payload substances comprise a surfactant. The surfactant of at least one first and/or second payload substance is primarily selected from standard surfactants known in the art to be suitable for contact with the skin. In some embodiments, the surfactant is at least one of: sodium cocoyl glutamate, cocamidopropyl betaine, sodium cocoyl glutamate, cocamidopropyl betaine, Disodium Lauryl Sulfosuccimate, decyl glucoside, coco betaine, lauryl glucoside, sodium methyl cocoyl taurate, decyl glucoside, coco glucoside, sodium methyl oleoyl taurate, Disodium Coco-glucoside Citrate, and mixtures thereof. In some embodiments, at least one of the first payload substance and/or second payload substances comprise a humectant. In some examples, the humectant includes panthenol.

In some embodiments, multilayered capsule **650** comprises at least one discreet inner layer **652** and at least one discreet outer layer **654.** In some embodiments, at least one discreet inner layer **652** of multilayered capsule **650** is essentially covered by at least one discreet outer layer **654** of multilayered capsule **650.**

In some embodiments, at least one payload substance, having a respectively higher value of an effect of a given concentration of at least one second payload substance in a target solution, on a dissolvability rate of at least one first payload substance in the target solution, with the given concentration of at least one second payload substance therein, incorporated within inner layer **652** of multilayered capsule **650.**

In some embodiments, at least one payload substance, having a respectively lower value of an effect of a given concentration of at least one first payload substance in a target solution, on a dissolvability rate of at least one second payload substance in the target solution, with the given concentration of at least one first payload substance therein, incorporated within outer layer **654** of multilayered capsule **650.**

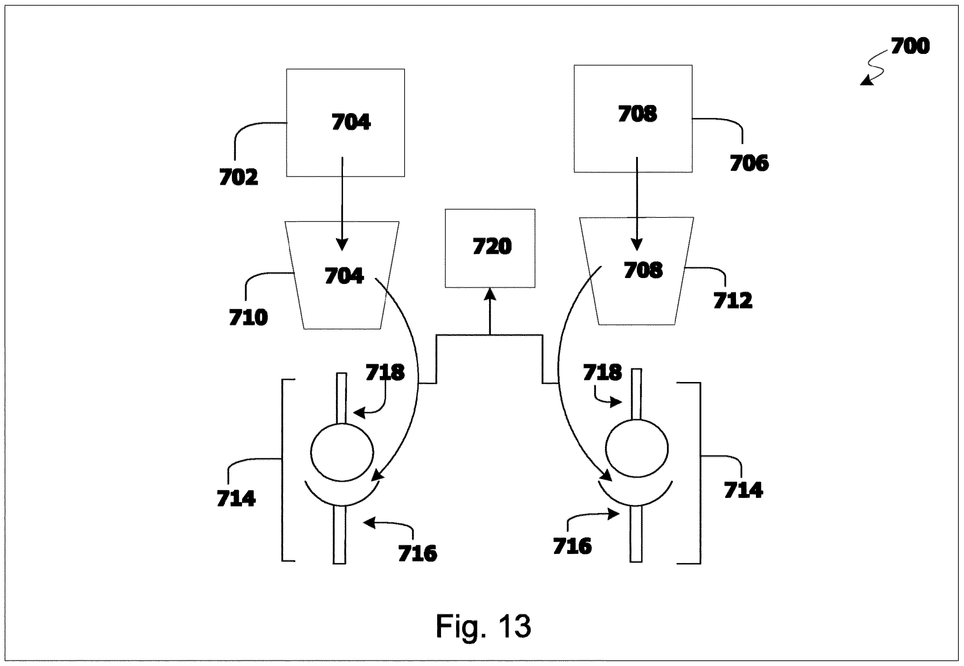

In accordance with some embodiments of the present invention, reference is now made **FIG 13****,** showing system **700** for manufacturing a dissolvable multilayered capsule, with a controllable arrangement of layers expediting a dissolvability rate thereof. The dissolvable multilayered capsule of the embodiment of **FIG 13** illustrates various features that may be interchangeable with elements of any other embodiment described in the specification.

In some embodiments, system **700** comprises container **702** with at least one first payload substance **704** to be dissolved in a target solution. In some embodiments, system **700** comprises container **706** with at least one second payload substance **708** to be dissolved in a target solution. Typically, at least one first payload substance **704** is distinctively different from at least one second payload substance **708.** In some embodiments, an effect of a given concentration of at least one second payload substance **708** in the target solution, on a dissolvability rate of at least one first payload substance **704** in the target solution, having the given concentration of at least one second payload substance **708** therein, is initially determined. In some embodiments, an effect of a given concentration of at least one first payload substance **704** in the target solution, on a dissolvability rate of at least one second payload substance **708** in the target solution, having the given concentration of at least one first payload substance **708** therein, is also thereafter determined. In some embodiments, system **700** further comprises first feeding hopper **710.** First feeding hopper **710** is operationally connected to container **702** with at least one first payload substance **704.** In some embodiments, system **700** further comprises second feeding hopper **712.** Second feeding hopper **712** is operationally connected to container **706** with at least one second payload substance **708.**

In some embodiments, system **700** further comprises fabricating module **714.** Typically, fabricating module **714** comprises at least one die part **716,** comprising a hollow section form, essentially matching an exterior contour of the multilayered capsule, configured to form a sequential operational connection, in turn, with first feeding hopper **710** and with second feeding hopper **712.** In some embodiments, fabricating module **714** of system **700** further comprises at least one movable punch part **718.** At least one movable punch part **718** comprises an elongated form, essentially matching an interior of the hollow section form of at least one die part **716.** At least one movable punch part **718** is configured to exsert a pressing force onto a payload substance in the hollow section form of at least one die part **716.**

In some embodiments, system **700** further comprises controller **720.** Controller **720** is configured to form the sequential operational connection of first feeding hopper **710** and in turn of second feeding hopper **712,** with the hollow section form of at least one die part **716,** in a predefined order. In some embodiments, a feeding hopper with at least one payload substance, having a respectively higher determined value of the effect of the given concentration of at least one second payload substance **708** in the target solution, on the dissolvability rate of at least one first payload substance **704** in the target solution, is activated first at the predefined order, thereby feeding into the hollow section form of at least one die part **716,** to fabricate the at least one payload substance of an inner layer of the multilayered capsule.

In some embodiments, a feeding hopper with at least one payload substance, having a respectively lower determined value of the effect of the given concentration of at least one first payload substance **704** in the target solution, on a dissolvability rate of at least one second payload substance **708** in the target solution, is activated second at the predefined order, thereby feeding into the hollow section form of at least one die part **716,** to fabricate the at least one payload substance of an outer layer of the multilayered capsule.

In some preferred embodiments the dissolvable multilayered capsule, with a controllable arrangement of layers expediting a dissolvability rate thereof, including any plurality of discrete layers, such as two, three, four or five discrete layers, arranged in accordance with the principals of the invention, as described hereinabove, to expedite the dissolvability rate of the multilayered capsule and the particular layers thereof.

### WORKING EXAMPLES

**Working Example 1** First working example of the dissolvable multilayered capsule, with controllable arrangement of layers thereof, reducing the dissolvability rate of the multilayered capsule is next described.

A target effect of reducing the dissolvability rate of the multilayered capsule in solvent solution of was predefined. A multilayered capsule was fabricated by forming an inner layer and an outer layer. The inner layer of the multilayered capsule was covered by the outer layer of the multilayered capsule. In order to reduce the dissolvability rate of the multilayered capsule in the solvent solution, a first payload substance of 0.1g of Sodium Gluconate was incorporated within the inner layer of the multilayered capsule. Then, a second payload substance of 0.3g of Xanthan gum was incorporated within the outer layer of the multilayered capsule. The solvent solution was 50ml of water for preparation of approximately 50ml of a cosmetic product.

**Working Example 2** Second working example of the dissolvable multilayered capsule, with controllable arrangement of layers thereof, increasing the dissolvability rate of the multilayered capsule is next described. A target effect of increasing the dissolvability rate of the multilayered capsule in solvent solution of was predefined. A multilayered capsule was fabricated by forming an inner layer and an outer layer. The inner layer of the multilayered capsule was covered by the outer layer of the multilayered capsule. In order to increase the dissolvability rate of the multilayered capsule in the solvent solution, a first payload substance of 0.3g of Xanthan gum was incorporated within the inner layer of the multilayered capsule. Then, a second payload substance of 0.1g of Sodium Gluconate was incorporated within the outer layer of the multilayered capsule. The solvent solution was 50ml of water for preparation of approximately 50ml of a cosmetic product.

**Working Example 3** Third working example of increasing the dissolvability rate of the multilayer capsule, as a function of particles size is next described. The selection of a particles sizes was made by either by ordering a substance with particles of a given standard size or by sieving the particles of a preselected size. Firstly, 1 gr tablet of sodium coco-sulfate was dissolved in 50 ml of distilled water in a warm temperature of 40 degrees Celsius, with pH of 7, where the tablet diameter was 22mm, the compression force was 30 bar and particle size of 300 micron. The time of dissolution obtained was 47 minutes. Secondly, 1 gr tablet of sodium coco-sulfate was dissolved in 50 ml of distilled water in a warm temperature of 40 degrees Celsius, with pH of 7, where the tablet diameter was 22mm, the compression force was 30 bar and particle size of 100 micron. The time of dissolution obtained was 39 minutes. The dissolution time was accelerated with smaller particle size.

**Working Example 4** Fourth working example of increasing the dissolvability rate of the multilayer capsule as a function of compression force is next described. The compression force was measured by pressure piston in bar units. Firstly, 1 gr tablet of sodium coco-sulfate was dissolved in 50 ml of distilled water in a warm temperature of 40 degrees Celsius, with pH of 7, where the tablet diameter was 22mm, the compression force was 30 bar and particle size of 100 micron. The time of dissolution obtained was 39 minutes. Secondly, 1 gr tablet of sodium coco-sulfate was dissolved in 50 ml of distilled water in a warm temperature of 40 degrees Celsius, with pH of 7, where the tablet diameter was 22mm, the compression force was 50 bar and particle size of 100 micron. The time of dissolution obtained was 49 minutes. The dissolution time was accelerated with smaller compression force.

## Claims

1. A method of manufacturing a dissolvable multilayered capsule (650), useful for effective preparation of a cosmetic product, with a controllable arrangement of layers, having an impact effect on the dissolvability and/or mixing thereof, based on a predetermined interaction between different payload substances incorporated in different layers of said capsule, to be dissolved in a common solvent, comprises the steps of:
(a) determining an impact effect of an interim solution having a given concentration of at least one first payload substance dissolved in a solvent, on a process of effective dissolution and/or mixing of at least one second payload substance in said interim solution, having said given concentration of said at least one first payload substance dissolved in said solvent;
wherein said at least one first payload substance is distinctively different from said at least one second payload substance;
(b) defining a desired target effect on an effective dissolution and/or mixing of said multilayered capsule (650) in said solvent;
(c) setting a prioritizing preference for said dissolution and/or mixing of said second payload substance in said interim solution, substantially after said first payload substance was dissolved in said solvent, over a dissolution and/or mixing of said first payload substance in an alternative interim solution, having said second payload substance dissolved in said solvent, based on said determining of said impact effect, at said step (a), and said defining of said desired target effect on said effective dissolution and/or mixing of said multilayered capsule (650) in said solvent, at said step (b);
(d) fabricating said multilayered capsule (650) comprising:
(I) forming at least one discreet inner layer (652) of said multilayered capsule (650);
(II) forming at least one discreet outer layer (654) of said multilayered capsule (650);
wherein said at least one discreet inner layer (652) of said multilayered capsule (650) is at least partially covered by said at least one discreet outer layer (654) of said multilayered capsule (650);
wherein said at least one first payload substance is incorporated either within said discreet inner layer (652) or within said discreet outer layer (654), based on said prioritizing preference, set at said step (c).

2. The method, as in claim 1, further comprises determining an impact effect of said alternative interim solution, having a given concentration of said at least one second payload substance dissolved in said solvent, on effective dissolution rate and/or mixing of said at least one first payload substance in said alternative interim solution, having said given concentration of said at least one second payload substance dissolved in said solvent.

3. The method, as in claim 1, wherein said target effect is selected from the group consisting of:
(I) reducing a dissolvability rate of said multilayered capsule (650) in said solvent, thereby prolonging a dissolution time of said multilayered capsule (650) in said solution, and
(II) increasing a dissolvability rate of said multilayered capsule (650) in said solvent, thereby shortening said dissolution time of said multilayered capsule (650) in said solvent.

4. The method, as in claim 1, further comprises furnishing said dissolvable multilayered capsule (650) with a coating configured to absorb and/or neutralize undesired additives or impurities in water.

5. The method, as in claim 1, further comprises furnishing said dissolvable multilayered capsule (650) with a coating comprising a water softening agent, including natrium chloride.

6. A dissolvable multilayered capsule (650), useful for effective preparation of a cosmetic product, with a controllable arrangement of layers, having an impact effect on the dissolvability and/or mixing thereof, based on a predetermined interaction between different payload substances incorporated in different layers of said capsule, to be dissolved in a common solvent, comprises:
(a) at least one first payload substance to be dissolved in a solvent, wherein an impact effect of an interim solution, having a given concentration of said at least one first payload substance dissolved in said solvent, on an effective dissolution rate and/or mixing of at least one second payload substance in said interim solution, having said given concentration of said at least one first payload substance dissolved in said solvent, is predetermined;
(b) at least one second payload substance to be dissolved in said interim solution;
wherein said at least one first payload substance is distinctively different from said at least one second payload substance;
(c) at least one discreet inner layer (652) of said multilayered capsule (650);
(d) at least one discreet outer layer (654) of said multilayered capsule (650);
wherein said at least one discreet inner layer (652) of said multilayered capsule (650) is essentially covered by said at least one discreet outer layer (654) of said multilayered capsule (650);
wherein a desired target effect on an effective dissolution and/or mixing of said multilayered capsule (650) in said solvent is predefined;
wherein a prioritizing preference for said dissolution and/or mixing of said second payload substance in said interim solution, substantially after said first payload substance was dissolved in said solvent, over a dissolution and/or mixing of said first payload substance in an alternative interim solution, having said second payload substance dissolved in said solvent, is preset, based on said determining of said impact effect, and said defining of said desired target effect on said effective dissolution and/or mixing of said multilayered capsule (650) in said solvent;
wherein said at least one first payload substance is incorporated either within said discreet inner layer (652) or within said discreet outer layer (654), based on said preset prioritizing preference.

7. The dissolvable multilayered capsule (650), as in claim 8, wherein an impact effect of said alternative interim solution, having a given concentration of said at least one second payload substance dissolved in said solvent, on effective dissolution rate and/or mixing of said at least one first payload substance in said alternative interim solution, having said given concentration of said at least one second payload substance dissolved in said solvent, is further predetermined.

8. The dissolvable multilayered capsule (650), as in claim 1, wherein said target effect is selected from the group consisting of:
(I) reducing a dissolvability rate of said multilayered capsule (650) in said solvent, thereby prolonging a dissolution time of said multilayered capsule in said solution, and
(II) increasing a dissolvability rate of said multilayered capsule (650) in said solvent, thereby shortening said dissolution time of said multilayered capsule (650) in said solvent.

9. The dissolvable multilayered capsule (650), as in claim 8, further comprises a coating configured to absorb and/or neutralize undesired additives or impurities in water.

10. The dissolvable multilayered capsule (650), as in claim 8, further comprises a coating comprising a water softening agent.

11. The dissolvable multilayered capsule (650), as in claim 8, further comprises a coating including natrium chloride.

12. A system (700) for manufacturing a dissolvable multilayered capsule (650), useful for effective preparation of a cosmetic product, with a controllable arrangement of layers, having an impact effect on the dissolvability and/or mixing thereof, based on a predetermined interaction between different payload substances incorporated in different layers of said capsule, to be dissolved in a common solvent, comprises:
(a) a container (702) with at least one first payload substance (704) to be dissolved in a solvent, wherein an impact effect of an interim solution, having a given concentration of said at least one first payload substance (704) dissolved in said solvent, on an effective dissolution rate and/or mixing of at least one second payload substance (708) in said interim solution, having said given concentration of said at least one first payload substance (704) dissolved in said solvent, is predetermined;
(b) a container (706) with at least one second payload substance (708) to be dissolved in said interim;
wherein said at least one first payload substance (704) is distinctively different from said at least one second payload substance (708);
wherein a desired target effect on an effective dissolution and/or mixing of said multilayered capsule (650) in said solvent is predefined;
wherein a prioritizing preference for said dissolution and/or mixing of said second payload substance (708) in said interim solution, substantially after said first payload substance (704) was dissolved in said solvent, over a dissolution and/or mixing of said first payload substance (704) in an alternative interim solution, having said second payload substance (708) dissolved in said solvent, is preset, based on said determining of said impact effect, and said defining of said desired target effect on said effective dissolution and/or mixing of said multilayered capsule (650) in said solvent;
(c) a first feeding hopper, operationally connected to said container (702) with said at least one first payload substance (704);
(d) a second feeding hopper, operationally connected to said container (706) with said at least one second payload substance (708);
(e) a fabricating module (714) comprising:
(I) at least one die part, comprising a hollow section form, essentially matching an exterior contour of said multilayered capsule (650), configured to form a sequential operational connection, in turn, with said first feeding hopper (710) and with said second feeding hopper (712);
(II) at least one movable punch part (718), comprising an elongated form, essentially matching an interior of said hollow section form of said at least one die part, configured to exsert a pressing force onto a payload substance in said hollow section form of said at least one die part;
(f) a controller (720) configured to form said sequential operational connection of said first feeding hopper, and in turn of said second feeding hopper, with said hollow section form of said at least one die part, in a defined order, wherein said at least one first payload substance (704) is incorporated either within said discreet inner layer (652) or within said discreet outer layer (654), based on said preset prioritizing preference.

13. The system (700), as in claim 12, wherein an impact effect of said alternative interim solution, having a given concentration of said at least one second payload substance (708) dissolved in said solvent, on effective dissolution rate and/or mixing of said at least one first payload substance (704) in said alternative interim solution, having said given concentration of said at least one second payload substance (708) dissolved in said solvent, is further predetermined.

14. The system (700), as in claim 12, wherein said target effect is selected from the group consisting of:
(I) reducing a dissolvability rate of said multilayered capsule (650) in said solvent, thereby prolonging a dissolution time of said multilayered capsule in said solution, and
(II) increasing a dissolvability rate of said multilayered capsule (650) in said solvent, thereby shortening said dissolution time of said multilayered capsule (650) in said solvent.

15. The system (700), as in claim 12, further comprises a pulverizator, configured for furnishing said dissolvable multilayered capsule (650) with a coating configured to absorb and/or neutralize undesired additives or impurities in water.
